# EUROPEAN PATENT APPLICATION

(11) **EP 4 512 799 A1**
(43) Date of publication of application: **26.02.2025**
(21) Application number: 23792201.8
(22) Date of filing: 20.04.2023
(51) Int. Cl.: C07C 323/22, C09K 11/02, H10K 85/60, H10K 59/38

(54) **COMPOUND, QUANTUM DOTS COORDINATED THEREWITH, COMPOSITION COMPRISING SAME, AND ELECTRONIC DEVICE MANUFACTURED USING SAME**

(30) Priority: 20.04.2022 KR 20220049103
(71) Applicant: Samsung Display Co., Ltd., Gyeonggi-do 17113 (KR)
(72) Inventor: KIM, Joon Hyung, Yongin-si Gyeonggi-do 17113 (KR); CHANG, Jae Bok, Yongin-si Gyeonggi-do 17113 (KR)
(74) Representative: Marks & Clerk LLP
(86) International application number: PCT/KR2023/005376
(87) International publication number: WO 2023/204627

(57) **Abstract**

The present application provides a compound, a quantum dot coordinated with the compound, a composition including the quantum dot coordinated with the compound, and an electronic apparatus manufactured with the composition. The compound comprises: a binding part comprising a dithio C₁-C₁₆ alkyl moiety; a hydrophilic linker comprising oxygen; and a terminal part comprising an unsubstituted C₆-C₄₀ aryl group, an unsubstituted C₂-C₁₀ alkyl group or an unsubstituted C₇-C₅₀ arylalkyl group. The binding part and the hydrophilic linker are linked by an ester linkage, and the hydrophilic linker connecting the binding portion and the end portion.

## Description

### Technical Field

The present disclosure relates to a compound, a quantum dot coordinated with the compound, a composition including the quantum dot coordinated with the compound, and an electronic apparatus manufactured with the composition.

### Background Art

Quantum dots are nanocrystals of semiconductor materials and exhibit a quantum confinement effect. Upon receiving light from an excitation source, quantum dots reach an energy excited state and then emit energy according to their corresponding energy band gap. Accordingly, even in quantum dots having the same compositional material, the emitted wavelength varies depending on the particle size. By adjusting the particle size of the quantum dots, light having a desired wavelength range as well as excellent color purity and/or high luminescence efficiency may be obtained due to the quantum confinement effect. Thus, quantum dots are applicable to various electronic devices, e.g., display devices.

### Disclosure

### Technical Problem

One or more embodiments relate to a ligand compound, a quantum dot coordinated with the compound, a composition including the quantum dot, and an electronic apparatus manufactured using the composition.

### Technical Solution

According to one or more embodiments, a compound may include:
a binding portion including a dithio C₁-C₁₆ alkyl moiety;
an end portion including an unsubstituted C₆-C₄₀ aryl group, an unsubstituted C₂-C₁₀ alkyl group, or an unsubstituted C₇-C₅₀ aryl alkyl group; and
a hydrophilic linker including oxygen, the hydrophilic linker connecting the binding portion and the end portion;
wherein the binding portion and the hydrophilic linker are connected by an ester linkage.

According to one or more embodiments, a quantum dot may be coordinated with the compound.

According to one or more embodiments, a composition may include
the quantum dot, and
a crosslinking monomer.

According to one or more embodiments, an electronic apparatus may include a light-emitting device including a first electrode, a second electrode facing the first electrode, and an interlayer arranged between the first electrode and the second electrode and including an emission layer,
a thin-film transistor including a source electrode and a drain electrode, and
a color conversion layer and/or color filter,
wherein the first electrode of the light-emitting device may be electrically connected to the source electrode or the drain electrode of the thin-film transistor, and the emission layer, the color conversion layer, and/or the color filter may include a layer manufactured using the composition.

### Advantageous Effects

Electronic apparatuses including light-emitting devices manufactured using the compositions including the ligand compound for the quantum dots according to an embodiment are excellent in efficiency.

### Description of Drawings

FIG. 1 is a schematic cross-sectional view of a light-emitting device according to an embodiment;
FIG. 2 is a cross-sectional view showing an electronic apparatus according to an embodiment of the present disclosure; and
FIG. 3 is a cross-sectional view showing an electronic apparatus according to another embodiment of the present disclosure.

### Best Mode

As a method of realizing color in a display device, a method of using a color filter that allows light having a select range of wavelengths to be emitted from the display has been widely used. Recently, a method has been proposed to further increase the color purity of the three primary colors of light, red, green, and blue, by combining a color conversion light-emitting material, which absorbs light of a specific wavelength and then converts and emits light of another wavelength with the use of a color filter.

In order to form a color conversion layer containing quantum dots, the quantum dots must be well dispersed in the resin polymer or monomer, which is a component of ink or a photoresist composition. Conventionally prepared quantum dots have a ligand layer on the surface, and the ligand layer immediately after preparation is made of oleic acid and lauric acid. Moreover, the ligand has a molecular structure made of non-polar hydrocarbons except for the surface binding site of the quantum dot, and thus, the ligand may be well dispersed in unsaturated hydrocarbon solvents such as n-hexane, and aromatic solvents such as chloroform and benzene. However, the dispersibility of the ligand is poor in polar solvents such as propylene glycol methyl ether acetate or a polar monomer such as (poly)acrylic acid.

A compound according to an aspect may include:
a binding portion including a dithio C₁-C₁₆ alkyl moiety;
an end portion including an unsubstituted C₆-C₄₀ aryl group, an unsubstituted C₂-C₁₀ alkyl group, or an unsubstituted C₇-C₄₀ aryl alkyl group and
a hydrophilic linker including oxygen, the hydrophilic linker connecting the binding position and the end portion, wherein the binding portion and the hydrophilic linker are connected by an ester linkage.

The binding portion and the end portion may be at opposite ends of the compound with the hydrophilic linker in between.

The dithio C₁-C₁₆ alkyl moiety included in the binding portion refers to an alkyl group to which two thiol groups are connected and having 1 to 16 carbon atoms.

The ester linkage refers to -COO- linkage. For example, the compound according to an embodiment may have a binding portion-COO-hydrophilic linker-end portion structure.

The binding portion of the compound according to an embodiment may have two thiol groups. Therefore, the quantum dot binding characteristics are excellent.

Because the hydrophilic linker of the compound according to an embodiment is hydrophilic linker containing oxygen, the hydrophilic linker has good miscibility with polar solvents or polar monomers.

The end portion of the compound according to an embodiment may not have a crosslinking function.

A compound according to an aspect may include:
a binding portion including a dithio C₁-C₁₆ alkyl moiety;
an end portion including an unsubstituted C₆-C₄₀ aryl group, an unsubstituted C₃-C₅₀ heteroaryl group, an unsubstituted C₂-C₁₀ alkyl group, an unsubstituted C₇-C₄₀ aryl alkyl group, a monovalent non-aromatic condensed polycyclic group, or a monovalent non-aromatic condensed heteropolycyclic group; and
a hydrophilic linker including oxygen, the hydrophilic linker connecting the binding position and the end portion, wherein the binding portion and the hydrophilic linker are connected by an ester linkage.

The binding portion and the end portion may be at opposite ends of the compound with the hydrophilic linker in between.

The compound according to an embodiment may act as a ligand to coordinate quantum dots. In the compound according to an embodiment, two thiol groups of the binding portion may be coordinated to the quantum dot. In a coordinate bond between the quantum dot and the thiol group, the bond between the quantum dot and the thiol group may be in a dynamic state of repeated formation and breaking.

In the case when there is only one thiol group, if the bonding between the quantum dot and the thiol group is broken, and if the distance between the quantum dot and the thiol group increases, it may be difficult to reform a bonding between the quantum dot and the thiol group.

However, if there are two thiol groups (e.g., thiol group A and thiol group B), even if the bonding between the quantum dot and one thiol group A is broken, if the bonding between the other thiol group B and the quantum dot is maintained, the bond that was broken between the quantum dot and thiol group A may more easily reform. This is because thiol group B is linked to thiol group A in the binding portion of the compound, and thus, even if the bonding between the quantum dot and the thiol group A is broken, a relatively small distance between the quantum dot and thiol group A is maintained, thus facilitating reformation of a bonding of thiol A with the quantum dot.

Because the quantum dots coordinated with the compound according to an embodiment includes a hydrophilic linker, the quantum dots may be well dispersed in a polar solvent or a polar monomer.

In the quantum dot coordinated with the compound according to an embodiment, the end portion of the compound may be located at an outermost portion, and the end portion may not have a crosslinking function.

Therefore, when the quantum dots coordinated with the compound according to an embodiment are crosslinked by mixing with a crosslinking monomer, the quantum dots may continue to have some fluidity or mobility within a polymerized matrix formed by the crosslinking monomer.

For example, the compound may be represented by Formula 1: 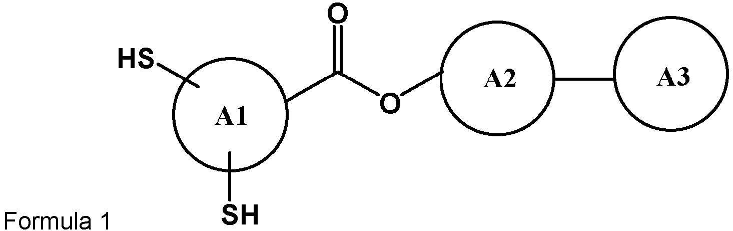 wherein, in Formula 1,
A1 indicates a C₁-C₁₆ alkyl moiety,
A2 indicates a hydrophilic linker; and
A3 indicates an end portion.

In an embodiment, an alkyl of the dithio C₁-C₁₆ alkyl moiety may be a linear or branched structure. For example, an alkyl of the C₁-C₁₆ alkyl moiety may have a linear structure.

In an embodiment, one thiol group in the dithio C₁-C₁₆ alkyl moiety may be positioned at a terminal carbon of the dithio C₁-C₁₆ alkyl moiety. For example, in a dithio C₁-C₁₆ alkyl moiety, one thiol group may be positioned at a terminal carbon of the dithio C₁-C₁₆ alkyl moiety and the other thiol group may be positioned at a different carbon of the dithio C₁-C₁₆ alkyl moiety.

In an embodiment, two to five carbons may be present between two thiol groups of a dithio C₁-C₁₆ alkyl moiety. For example, two or three carbons may be present between two thiol groups of the dithio C₁-C₁₆ alkyl moiety.

In an embodiment, the hydrophilic linker including oxygen may be a linker including one or more ethylene glycol units and/or one or more propylene glycol units. For example, the number of the one or more ethylene glycol units, or the number of the one or more propylene glycol units, may each independently be 1 to 10.

In an embodiment, the unsubstituted C₆-C₄₀ aryl group may include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, an ovalenyl group, or any combination thereof.

In an embodiment, the unsubstituted C₂-C₁₀ alkyl group may include an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or any combination thereof.

The unsubstituted C₇-C₅₀ aryl alkyl group, indicates -A₁₀₄A₁₀₅ (here, A₁₀₄ is a C₁-C₁₀ alkylene group, and A₁₀₅ is a C₆-C₄₀ aryl group). For more details on the alkylene group, related descriptions provided herein may be referred to.

In an embodiment, the compound may include one of the compounds below.

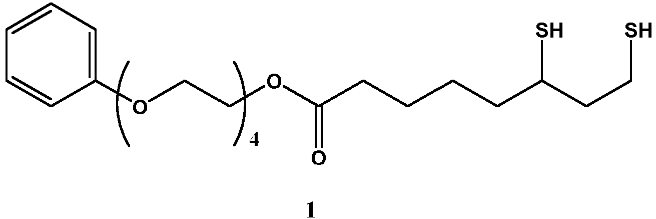

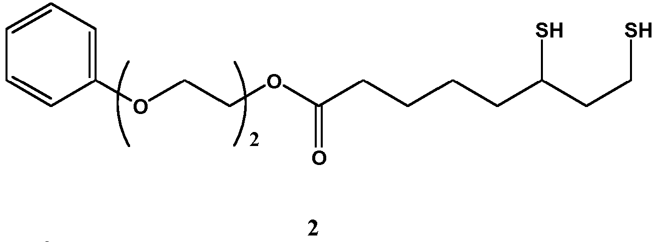

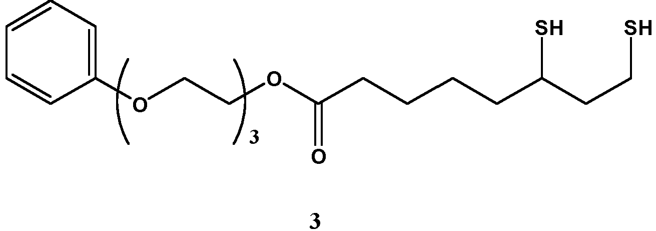

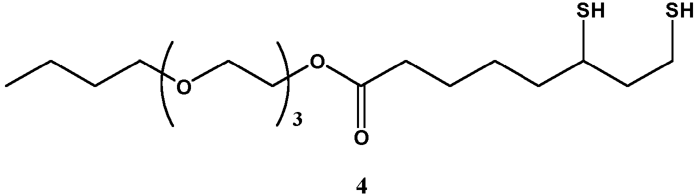

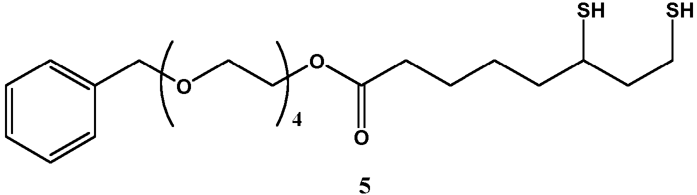

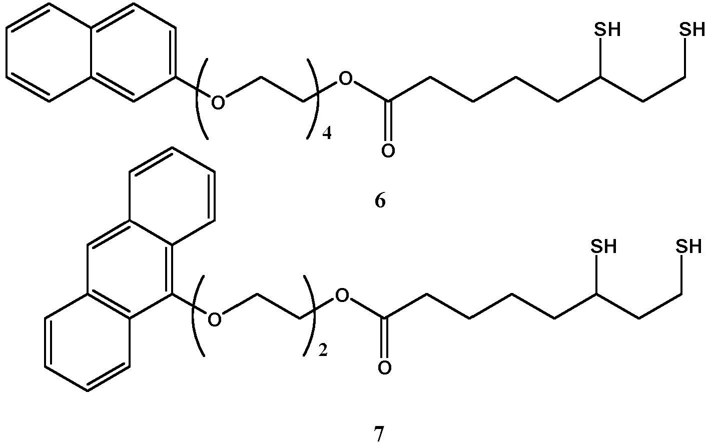

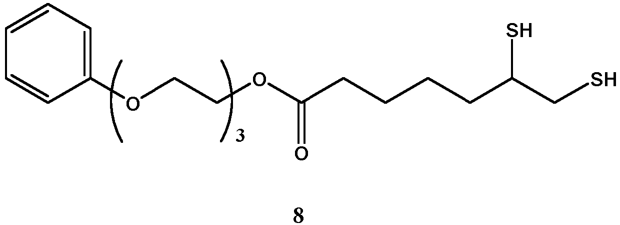

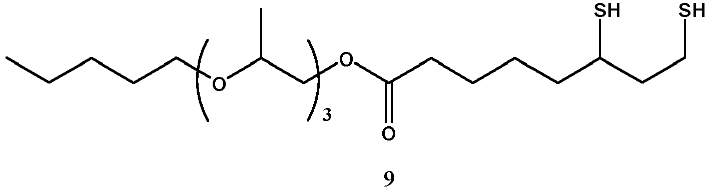

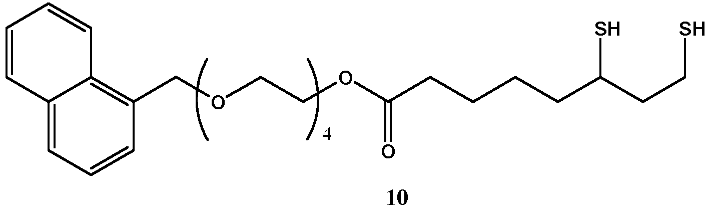

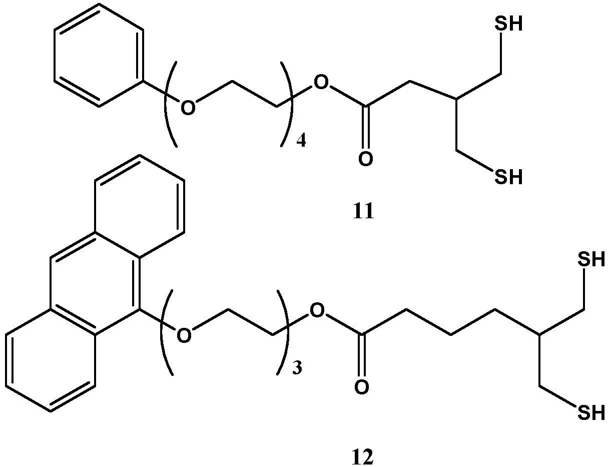

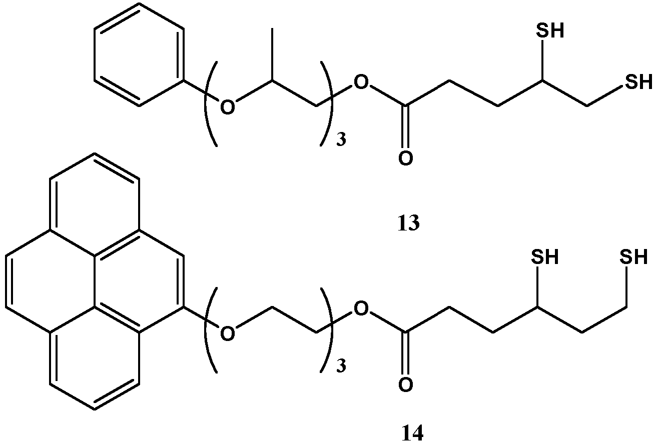

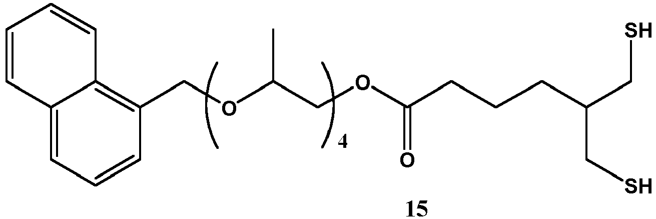

The quantum dot according to another aspect may be a quantum dot coordinated with the compound as described herein.

For example, because the compound has two thiol groups in the binding portion, the binding force between the compound and the quantum dot may increase, thereby increasing the stability of the quantum dot.

A composition according to another embodiment may include the quantum dot and a crosslinking monomer.

A weight ratio of the quantum dot to the crosslinking monomer may be about 1:0.5 to about 1:2.

In an embodiment, the composition may further include a photoinitiator. For example, the photoinitiator may include diphenyl(2,4,6-trimethylbenzoyl)phosphine oxide, 4-acryloxybenzophenone, 2,2-dimethoxy-2-phenylacetophenone, 2-hydroxy-2-methyl-1-phenyl-1-propane-1-one, ethyl(2,4,6-trimethylbenzoyl)phenyl phosphinate, bisacrylphosphine oxide, or any combination thereof.

In an embodiment, the crosslinking monomer may be an acrylic monomer.

For example, the crosslinking monomer may include 1,6-hexanediol diacrylate, 2-ethylhexyl (meth)acrylate, ethyl (meth) acrylate, methyl (meth)acrylate, n-propyl (meth)acrylate, isopropyl (meth)acrylate, pentyl (meth)acrylate, n-octyl (meth)acrylate, isooctyl (meth)acrylate, isononyl (meth)acrylate, n-butyl (meth)acrylate, isobutyl (meth) acrylate, n-hexyl (meth)acrylate, n-nonyl (meth)acrylate, isoamyl (meth)acrylate, n-decyl (meth)acrylate, isodecyl (meth)acrylate, dodecyl (meth)acrylate, isobornyl (meth)acrylate, cyclohexyl (meth)acrylate, phenyl (meth)acrylate, benzyl (meth)acrylate, isostearyl (meth)acrylate, 2-methylbutyl (meth)acrylate, or any combination thereof.

A quantum dot is a semiconductor nanomaterial having a particle size of several nanometers to several hundreds of nanometers and may include a core including a material having a small band gap and a shell around the core.

In an embodiment, the quantum dot may have a core-shell structure including: a core including a semiconductor compound; and a shell including an oxide of a metal, a metalloid or a non-metal, a semiconductor compound, or a combination thereof.

Details of the semiconductor compound and the oxide of the metal, the metalloid, or the non-metal are described below.

In an embodiment, the viscosity (at 25 °C) of the composition may be about 2 centipoise (cP) to about 30 cP.

When the viscosity is within the above range, there is no difficulty in forming a layer with the composition according to an embodiment using a solution process, for example, by spin coating method or an inkjet printing method.

### First Electrode 110

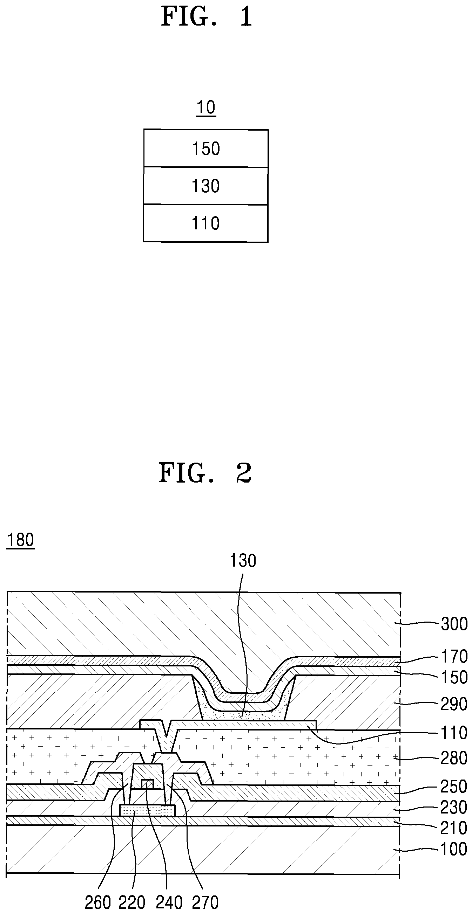

FIG. 1 is a schematic cross-sectional view of a light-emitting device 10 according to an embodiment. The light-emitting device 10 includes a first electrode 110, an interlayer 130, and a second electrode 150.

In FIG. 1, a substrate may be additionally located under the first electrode 110 or on the second electrode 150. As the substrate, a glass substrate or a plastic substrate may be used. In one or more embodiments, the substrate may be a flexible substrate, and may include plastics with excellent heat resistance and durability, such as polyimide, polyethylene terephthalate (PET), polycarbonate, polyethylene naphthalate, polyarylate (PAR), polyetherimide, or any combination thereof.

The first electrode 110 may be formed by, for example, depositing or sputtering a material for forming the first electrode 110 on an upper portion of the substrate. When the first electrode 110 is an anode, a material for forming the first electrode 110 may be a high-work function material that facilitates injection of holes.

The first electrode 110 may be a reflective electrode, a semi-transmissive electrode, or a transmissive electrode. When the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. In one or more embodiments, when the first electrode 110 is a semi-transmissive electrode or a reflective electrode, a material for forming the first electrode 110 may include magnesium (Mg), silver (Ag), aluminium (Al), aluminium-lithium (Al-Li), calcium (Ca), magnesium-indium (Mg-In), magnesium-silver (Mg-Ag), or any combination thereof.

The first electrode 110 may have a single-layered structure consisting of a single layer or a multi-layered structure including a plurality of layers. For example, the first electrode 110 may have a three-layered structure of ITO/Ag/ITO.

The interlayer 130 may be located on the first electrode 110. The interlayer 130 may include an emission layer.

The interlayer 130 may further include a hole transport region located between the first electrode 110 and the emission layer, and an electron transport region located between the emission layer and the second electrode 150.

The interlayer 130 may further include, in addition to various organic materials, a metal-containing compound such as an organometallic compound, an inorganic material such as quantum dots, or the like.

In one or more embodiments, the interlayer 130 may include, i) two or more emitting units sequentially stacked between the first electrode 110 and the second electrode 150, and ii) a charge generation layer located between the two or more emitting units. When the interlayer 130 includes emitting units and a charge generation layer as described above, the light-emitting device 10 may be a tandem light-emitting device.

The hole transport region may have: i) a single-layered structure consisting of a single layer consisting of a single material, ii) a single-layered structure consisting of a single layer consisting of a plurality of different materials, or iii) a multi-layered structure including a plurality of layers including different materials.

The hole transport region may include a hole injection layer, a hole transport layer, an emission auxiliary layer, an electron-blocking layer, or any combination thereof.

For example, the hole transport region may have a multi-layered structure including a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, the layers of each structure being stacked sequentially from the first electrode 110.

The hole transport region may include a compound represented by Formula 201, a compound represented by Formula 202, or any combination thereof: 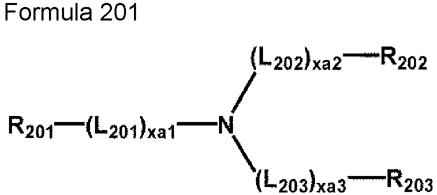 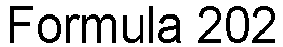 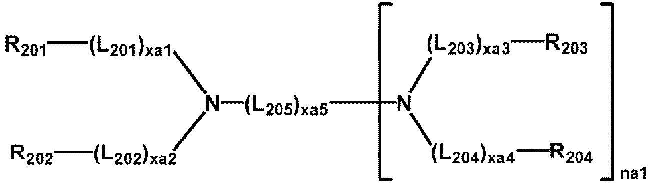 wherein, in Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer selected from 0 to 5,
xa5 may be an integer selected from 1 to 10,
R₂₀₁ to R₂₀₄ and Q₂₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group (for example, a carbazole group or the like) unsubstituted or substituted with at least one R₁₀ₐ (for example, Compound HT16),
R₂₀₃ and R₂₀₄ may optionally be linked to each other, via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group unsubstituted or substituted with at least one R₁₀ₐ, and
na1 may be an integer selected from 1 to 4.

For example, each of Formulae 201 and 202 may include at least one of groups represented by Formulae CY201 to CY217: 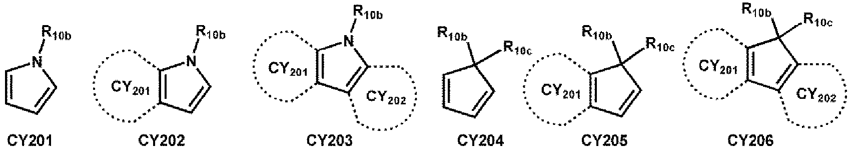 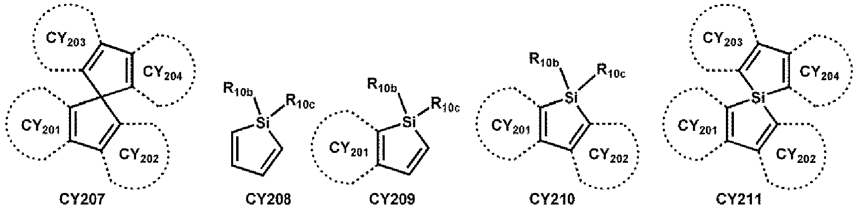 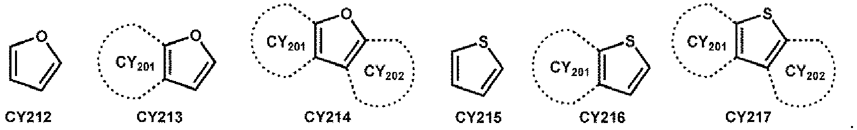 R_{10b} and R_{10c} in Formulae CY201 to CY217 are the same as described in connection with R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ.

In one or more embodiments, ring CY₂₀₁ to ring CY₂₀₄ in Formulae CY201 to CY217 may each independently be a benzene group, a naphthalene group, a phenanthrene group, or an anthracene group.

In one or more embodiments, each of Formulae 201 and 202 may include at least one of groups represented by Formulae CY201 to CY203.

In one or more embodiments, Formula 201 may include at least one of the groups represented by Formulae CY201 to CY203 and at least one of the groups represented by Formulae CY204 to CY217.

In one or more embodiments, in Formula 201, xa1 may be 1, R₂₀₁ may be a group represented by one of Formulae CY201 to CY203, xa2 may be 0, and R₂₀₂ may be a group represented by one of Formulae CY204 to CY207.

In one or more embodiments, each of Formulae 201 and 202 may not include a group represented by one of Formulae CY201 to CY203.

In one or more embodiments, each of Formulae 201 and 202 may not include a group represented by one of Formulae CY201 to CY203, and may include at least one of the groups represented by Formulae CY204 to CY217.

In one or more embodiments, each of Formulae 201 and 202 may not include a group represented by one of Formulae CY201 to CY217.

In an embodiment, the hole transport region may include one of Compounds HT1 to HT46, m-MTDATA, TDATA, 2-TNATA, NPB(NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), or any combination thereof:

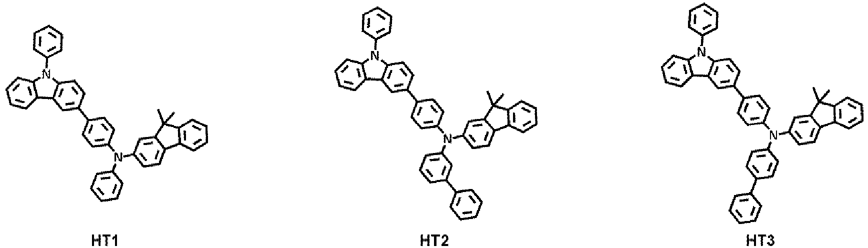

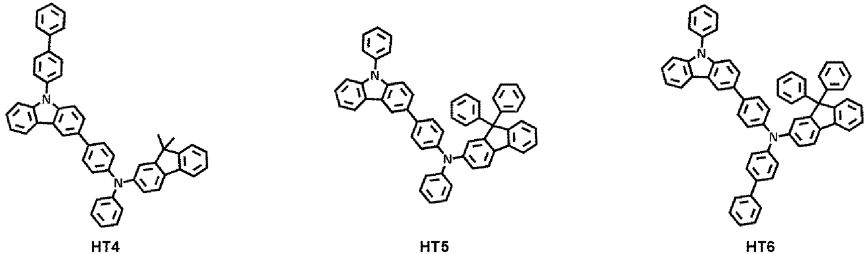

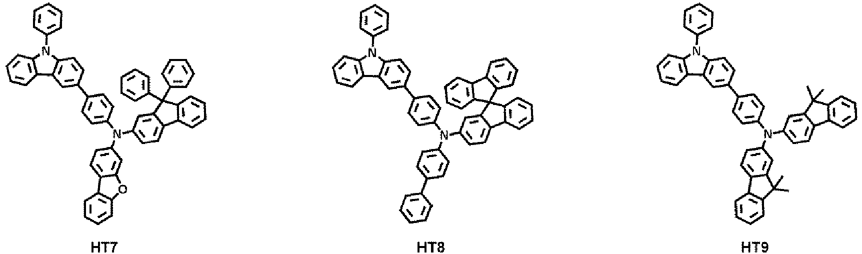

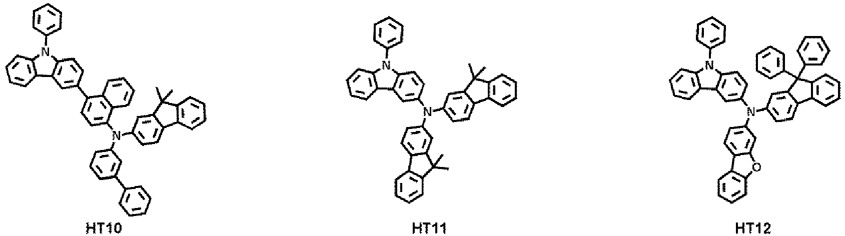

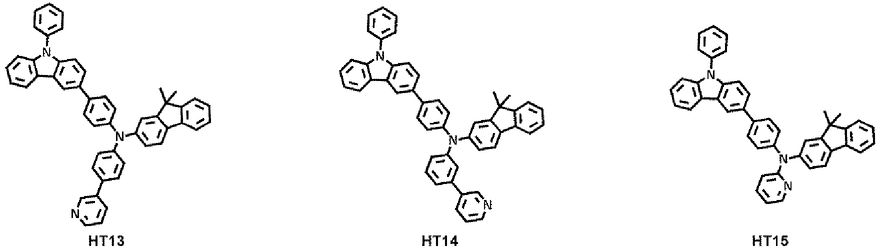

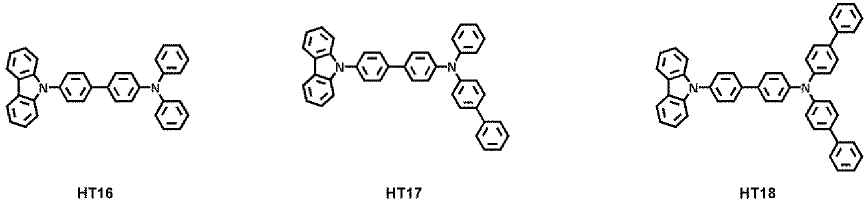

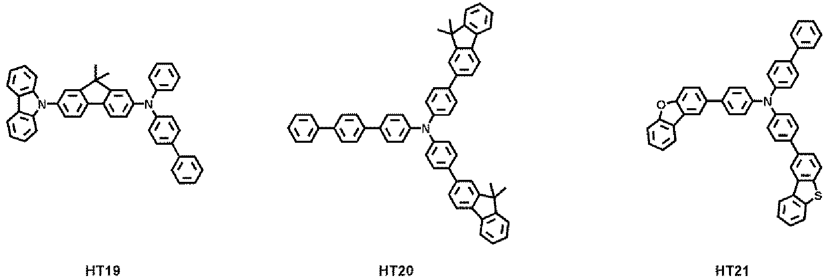

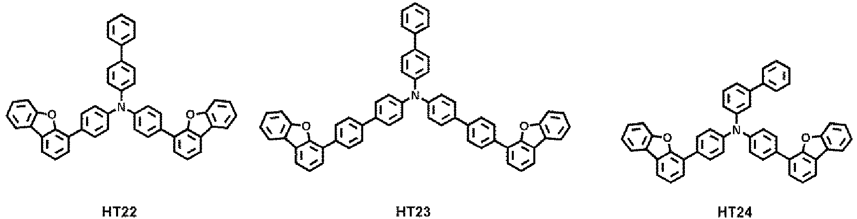

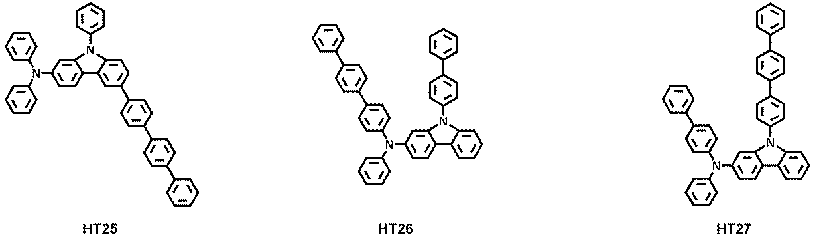

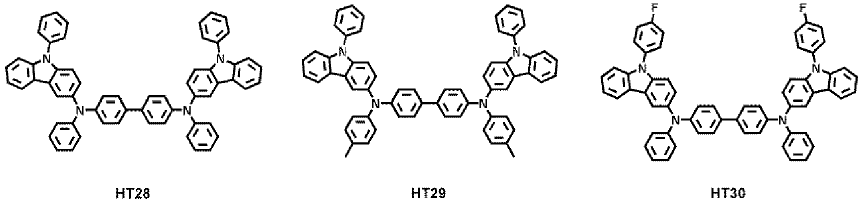

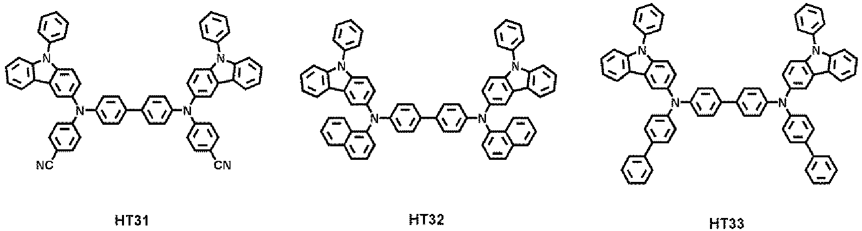

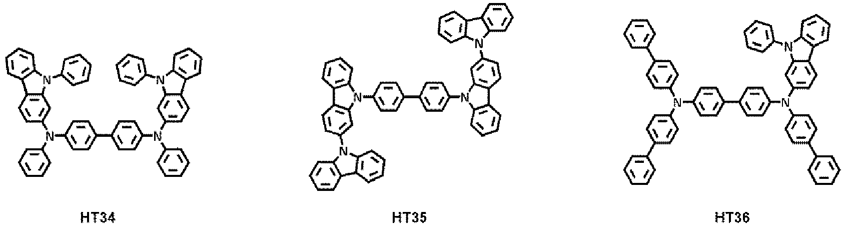

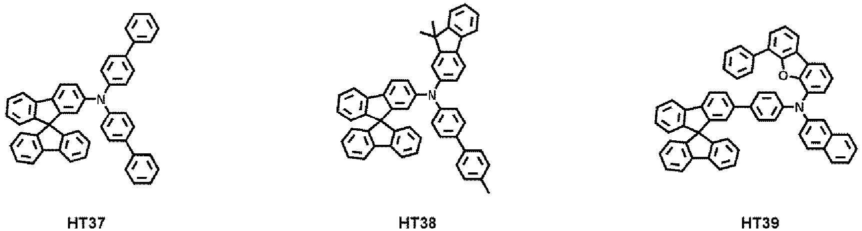

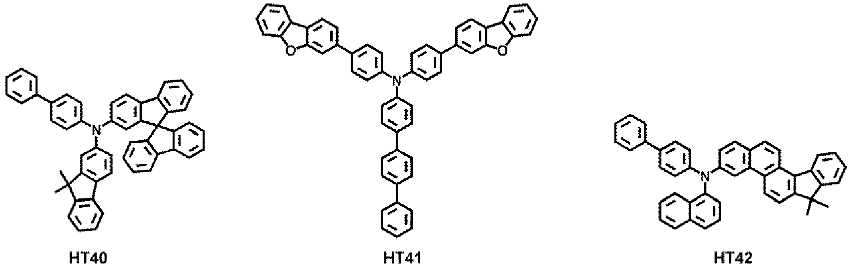

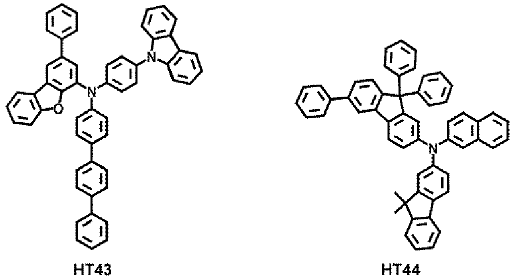

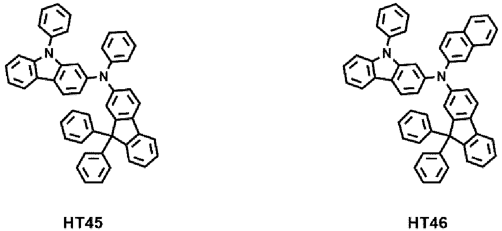

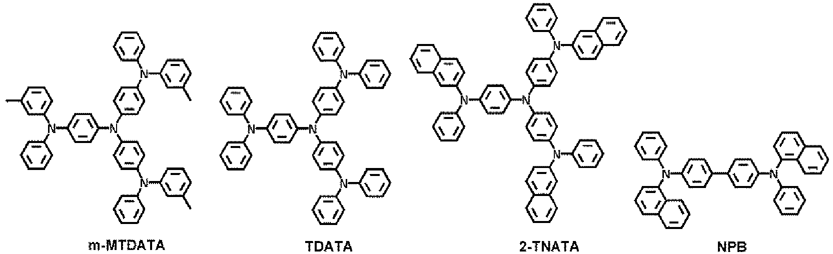

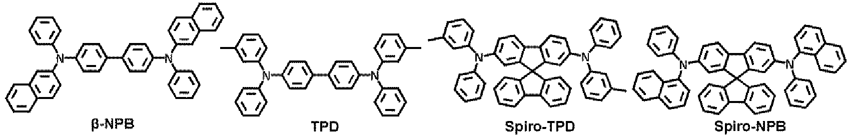

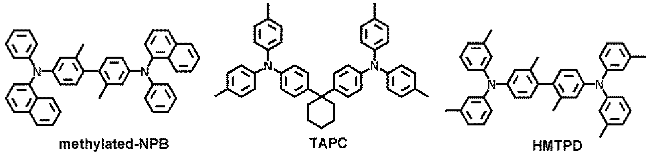

A thickness of the hole transport region may be in a range of about 50 Å to about 10,000 Å, for example, about 100 Å to about 4,000 Å. When the hole transport region includes a hole injection layer, a hole transport layer, or any combination thereof, a thickness of the hole injection layer may be in a range of about 100 Å to about 9,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer, and the hole transport layer are within these ranges, satisfactory hole transporting characteristics may be obtained without a substantial increase in driving voltage.

The emission auxiliary layer may increase light-emission efficiency by compensating for an optical resonance distance according to the wavelength of light emitted from the emission layer, and the electron-blocking layer may block the leakage of electrons from the emission layer to the hole transport region. Materials that may be included in the hole transport region may be included in the emission auxiliary layer and the electron-blocking layer.

The hole transport region may further include, in addition to these materials, a charge-generation material for the improvement of conductive properties. The charge-generation material may be uniformly or non-uniformly dispersed in the hole transport region (for example, in the form of a single layer consisting of a charge-generation material).

The charge-generation material may be, for example, a p-dopant.

For example, the lowest unoccupied molecular orbital (LUMO) energy level of the p-dopant may be -3.5 electron volts (eV) or less.

In one or more embodiments, the p-dopant may include a quinone derivative, a cyano group-containing compound, a compound including element EL1 and element EL2, or any combination thereof.

Examples of the quinone derivative are TCNQ, F4-TCNQ, etc.

Examples of the cyano group-containing compound are HAT-CN, and a compound represented by Formula 221 below. 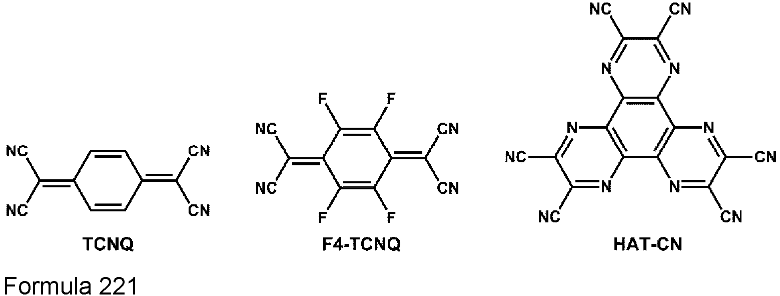 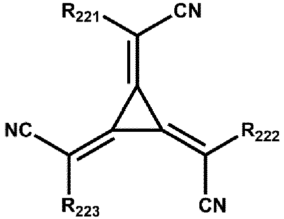 In Formula 221,
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with a cyano group; -F; -Cl; -Br; -I; a C₁-C₂₀ alkyl group substituted with a cyano group, -F, -Cl, -Br, -I, or any combination thereof; or any combination thereof.

In the compound including element EL1 and element EL2, element EL1 may be metal, metalloid, or any combination thereof, and element EL2 may be non-metal, metalloid, or any combination thereof.

Examples of the metal are an alkali metal (for example, lithium (Li), sodium (Na), potassium (K), rubidium (Rb), cesium (Cs), etc.); alkaline earth metal (for example, beryllium (Be), magnesium (Mg), calcium (Ca), strontium (Sr), barium (Ba), etc.); transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); post-transition metal (for example, zinc (Zn), indium (In), tin (Sn), etc.); and lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.).

Examples of the metalloid are silicon (Si), antimony (Sb), and tellurium (Te).

Examples of the non-metal are oxygen (O) and halogen (for example, F, Cl, Br, I, etc.).

Examples of the compound including element EL1 and element EL2 are metal oxide, metal halide (for example, metal fluoride, metal chloride, metal bromide, or metal iodide), metalloid halide (for example, metalloid fluoride, metalloid chloride, metalloid bromide, or metalloid iodide), metal telluride, or any combination thereof.

Examples of the metal oxide are tungsten oxide (for example, WO, W₂O₃, WO₂, WO₃, W₂O₅, etc.), vanadium oxide (for example, VO, V₂O₃, VO₂, V₂O₅, etc.), molybdenum oxide (for example, MoO, Mo₂O₃, MoO₂, MoO₃, Mo₂O₅, etc.), and rhenium oxide (for example, ReO₃, etc.).

Examples of the metal halide are alkali metal halide, alkaline earth metal halide, transition metal halide, post-transition metal halide, and lanthanide metal halide.

Examples of the alkali metal halide may include LiF, NaF, KF, RbF, CsF, LiCl, NaCl, KCI, RbCl, CsCl, LiBr, NaBr, KBr, RbBr, CsBr, Lil, Nal, KI, Rbl, and Csl.

Examples of the alkaline earth metal halide are BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, and BaI₂.

Examples of the transition metal halide are titanium halide (for example, TiF₄, TiCl₄, TiBr₄, TiI₄, etc.), zirconium halide (for example, ZrF₄, ZrCl₄, ZrBr₄, ZrI₄, etc.), hafnium halide (for example, HfF₄, HfCl₄, HfBr₄, HfI₄, etc.), vanadium halide (for example, VF₃, VCl₃, VBr₃, VI₃, etc.), niobium halide (for example, NbF₃, NbCl₃, NbBr₃, NbI₃, etc.), tantalum halide (for example, TaF₃, TaCl₃, TaBr₃, TaI₃, etc.), chromium halide (for example, CrF₃, CrCl₃, CrBr₃, CrI₃, etc.), molybdenum halide (for example, MoF₃, MoCl₃, MoBr₃, MoI₃, etc.), tungsten halide (for example, WF₃, WCl₃, WBr₃, WI₃, etc.), manganese halide (for example, MnF₂, MnCl₂, MnBr₂, MnI₂, etc.), technetium halide (for example, TcF₂, TcCl₂, TcBr₂, TcI₂, etc.), rhenium halide (for example, ReF₂, ReCl₂, ReBr₂, ReI₂, etc.), ferrous halide (for example, FeF₂, FeCl₂, FeBr₂, FeI₂, etc.), ruthenium halide (for example, RuF₂, RuCl₂, RuBr₂, RuI₂, etc.), osmium halide (for example, OsF₂, OsCl₂, OsBr₂, OsI₂, etc.), cobalt halide (for example, CoF₂, CoCl₂, CoBr₂, CoI₂, etc.), rhodium halide (for example, RhF₂, RhCl₂, RhBr₂, RhI₂, etc.), iridium halide (for example, IrF₂, IrCl₂, IrBr₂, IrI₂, etc.), nickel halide (for example, NiF₂, NiCl₂, NiBr₂, NiI₂, etc.), palladium halide (for example, PdF₂, PdCl₂, PdBr₂, PdI₂, etc.), platinum halide (for example, PtF₂, PtCl₂, PtBr₂, PtI₂, etc.), cuprous halide (for example, CuF, CuCl, CuBr, Cul, etc.), silver halide (for example, AgF, AgCl, AgBr, Agl, etc.), and gold halide (for example, AuF, AuCl, AuBr, Aul, etc.).

Examples of the post-transition metal halide are zinc halide (for example, ZnF₂, ZnCl₂, ZnBr₂, Znl₂, etc.), indium halide (for example, InI₃, etc.), and tin halide (for example, SnI₂, etc.).

Examples of the lanthanide metal halide are YbF, YbF₂, YbF₃, SmF₃, YbCl, YbCl₂, YbCl₃, SmCl₃, YbBr, YbBr₂, YbBr₃, SmBr₃, Ybl, YbI₂, YbI₃, and SmI₃.

An example of the metalloid halide is antimony halide (for example, SbCl₅, etc.).

Examples of the metal telluride are alkali metal telluride (for example, Li₂Te, Na₂Te, K₂Te, Rb₂Te, Cs₂Te, etc.), alkaline earth metal telluride (for example, BeTe, MgTe, CaTe, SrTe, BaTe, etc.), transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), post-transition metal telluride (for example, ZnTe, etc.), and lanthanide metal telluride (for example, LaTe, CeTe, PrTe, NdTe, PmTe, EuTe, GdTe, TbTe, DyTe, HoTe, ErTe, TmTe, YbTe, LuTe, etc.).

### Emission layer in interlayer 130

When the light-emitting device 10 is a full-color light-emitting device, the emission layer may be patterned into a red emission layer, a green emission layer, and/or a blue emission layer, according to a sub-pixel. In one or more embodiments, the emission layer may have a stacked structure of two or more layers of a red emission layer, a green emission layer, and a blue emission layer, in which the two or more layers contact each other or are separated from each other to emit white light. In one or more embodiments, the emission layer may include two or more materials of a red light-emitting material, a green light-emitting material, and a blue light-emitting material, in which the two or more materials are mixed with each other in a single layer to emit white light.

The emission layer may include a host and a dopant. The dopant may include a phosphorescent dopant, a fluorescent dopant, or any combination thereof.

The amount of the dopant in the emission layer may be from about 0.01 parts by weight to about 15 parts by weight based on 100 parts by weight of the host of the emission layer.

In one or more embodiments, the emission layer may include the quantum dots.

Meanwhile, the emission layer may include a delayed fluorescence material. The delayed fluorescence material may act as a host or a dopant in the emission layer.

A thickness of the emission layer may be in a range of about 100 Å to about 1,000 Å, for example, about 200 Å to about 600 Å. When the thickness of the emission layer is within these ranges, excellent light-emission characteristics may be obtained without a substantial increase in driving voltage.

### Quantum dot

The emission layer may include quantum dots.

The term "quantum dots" as used herein refers to crystals of a semiconductor compound and may include any material capable of emitting light of various emission wavelengths according to the particle size of the crystals.

A diameter of the quantum dot may be, for example, in a range of about 1 nm to about 10 nm.

The quantum dot may be synthesized by a wet chemical process, a metal organic chemical vapor deposition (MOCVD) process, a molecular beam epitaxy (MBE) process, or any process similar thereto.

The wet chemical process is a method including mixing a precursor material with an organic solvent and then growing a quantum dot particle crystal. When the quantum dot particle crystal grows, the organic solvent naturally acts as a dispersant coordinated on the surface of the quantum dot particle crystal and controls the growth of the quantum dot particle crystal so that the growth of quantum dot particle crystals can be controlled with a process that is lower in costs, and is easier than vapor deposition methods, such as metal organic chemical vapor deposition (MOCVD) or molecular beam epitaxy (MBE).

The quantum dot may include Group II-VI semiconductor compounds, Group III-V semiconductor compounds, Group III-VI semiconductor compounds, Group I-III-VI semiconductor compounds, Group IV-VI semiconductor compounds, a Group IV element or compound, or any combination thereof.

Examples of the Group II-VI semiconductor compound are a binary compound, such as CdS, CdSe, CdTe, ZnS, ZnSe, ZnTe, ZnO, HgS, HgSe, HgTe, MgSe, or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, or MgZnS; a quaternary compound, such as CdZnSeS, CdZnSeTe, CdZnSTe, CdHgSeS, CdHgSeTe, CdHgSTe, HgZnSeS, HgZnSeTe, or HgZnSTe; or any combination thereof.

Examples of the Group III-V semiconductor compound may include: a binary compound, such as GaN, GaP, GaAs, GaSb, AIN, AlP, AlAs, AlSb, InN, InP, InAs, or InSb; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, or InPSb; a quaternary compound, such as GaAlNP, GaAINAs, GaAINSb, GaAlPAs, GaAlPSb, GalnNP, GalnNAs, GaInNSb, GalnPAs, GalnPSb, InAlNP, InAlNAs, InAlNSb, InAlPAs, or InAlPSb; or any combination thereof. Meanwhile, the Group III-V semiconductor compound may further include a Group II element. Examples of the Group III-V semiconductor compound further including a Group II element are InZnP, InGaZnP, InAlZnP, etc.

Examples of the Group III-VI semiconductor compound are: a binary compound, such as GaS, GaSe, Ga₂Se₃, GaTe, InS, InSe, In₂S₃, In₂Se₃, or InTe; a ternary compound, such as InGaS₃, or InGaSe₃; or any combination thereof.

Examples of the Group I-III-VI semiconductor compound are: a ternary compound, such as AgInS, AgInS₂, CulnS, CuInS₂, CuGaO₂, AgGaO₂, or AgAlO₂; or any combination thereof.

Examples of the Group IV-VI semiconductor compound are: a binary compound, such as SnS, SnSe, SnTe, PbS, PbSe, or PbTe; a ternary compound, such as SnSeS, SnSeTe, SnSTe, PbSeS, PbSeTe, PbSTe, SnPbS, SnPbSe, or SnPbTe; a quaternary compound, such as SnPbSSe, SnPbSeTe, or SnPbSTe; or any combination thereof.

The Group IV element or compound may include: a single element, such as Si or Ge; a binary compound, such as SiC or SiGe; or any combination thereof.

Each element included in a multi-element compound such as the binary compound, the ternary compound, and the quaternary compound may be present at a uniform concentration or non-uniform concentration in a particle.

Meanwhile, the quantum dot may have a single structure in which the concentration of each element in the quantum dot is uniform, or a core-shell dual structure. For example, the material included in the core and the material included in the shell may be different from each other.

The shell of the quantum dot may act as a protective layer that prevents chemical degeneration, e.g., oxidation, of the core to maintain semiconductor characteristics, and/or as a charging layer that imparts electrophoretic characteristics to the quantum dot. The shell may be a single layer or a multi-layer. The interface between the core and the shell may have a concentration gradient in which the concentration of an element existing in the shell decreases toward the center of the core.

Examples of the shell of the quantum dot may be an oxide of a metal, a metalloid, or a non-metal, a semiconductor compound, or any combination thereof. Examples of the oxide of metal, metalloid, or non-metal are a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, or NiO; a ternary compound, such as MgAl₂O₄, CoFe₂O₄, NiFe₂O₄, or CoMn₂O₄; or any combination thereof. Examples of the semiconductor compound are, as described herein, a Group II-VI semiconductor compound; a Group III-V semiconductor compound; a Group III-VI semiconductor compound; a Group I-III-VI semiconductor compound; a Group IV-VI semiconductor compound; or any combination thereof. For example, the semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, or any combination thereof.

A full width at half maximum (FWHM) of the emission wavelength spectrum of the quantum dot may be about 45 nm or less, for example, about 40 nm or less, for example, about 30 nm or less, and within these ranges, color purity or color reproducibility may be increased.. In addition, since the light emitted through the quantum dot is emitted in all directions, the wide viewing angle may be improved.

In addition, the quantum dot may be in the form of a spherical nanoparticle, a pyramidal nanoparticle, a multi-arm nanoparticle, a cubic nanoparticle, a nanotube, a nanowire, a nanofiber, or a nanoplate.

Since the energy band gap may be adjusted by controlling the size of the quantum dot, light having various wavelength bands may be obtained from the quantum dot emission layer. Accordingly, by using quantum dots of different sizes, a light-emitting device that emits light of various wavelength bands may be implemented. In one or more embodiments, the size of the quantum dot may be selected to emit red, green and/or blue light. In addition, the size of the quantum dot may be configured to emit white light by combination of light of various colors.

### Electron transport region in interlayer 130

The electron transport region may have: i) a single-layered structure consisting of a single layer consisting of a single material, ii) a single-layered structure consisting of a single layer consisting of a plurality of different materials, or iii) a multi-layered structure including a plurality of layers including different materials.

The electron transport region may include a hole-blocking layer, an electron transport layer, an electron injection layer, or any combination thereof.

In an embodiment, the electron transport region may have an electron transport layer/electron injection layer structure or a hole-blocking layer/electron transport layer/electron injection layer structure, wherein, in each structure, constituting layers are sequentially stacked from the emission layer.

In an embodiment, the electron transport region (for example, the hole-blocking layer, or the electron transport layer in the electron transport region) may include a metal-free compound including at least one π electron-deficient nitrogen-containing C₁-C₆₀ cyclic group.

For example, the electron transport region may include a compound represented by Formula 601 below:

Formula 601 [Ar₆₀₁]ₓₑ₁₁-(L₆₀₁)ₓₑ₁-R₆₀₁]ₓₑ₂₁

wherein, in Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or -P(=O)(Q₆₀₁)(Q₆₀₂),
Q₆₀₁ to Q₆₀₃ may each be the same as described herein with respect to Q₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group unsubstituted or substituted with at least one R₁₀ₐ.

For example, when xe11 in Formula 601 is 2 or more, two or more of Ar₆₀₁(s) may be linked to each other via a single bond.

In other embodiments, Ar₆₀₁ in Formula 601 may be a substituted or unsubstituted anthracene group unsubstituted or substituted with at least one R₁₀ₐ.

In other embodiments, the electron transport region may include a compound represented by Formula 601-1: 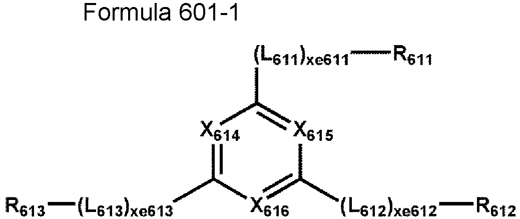 wherein, in Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may be N,
L₆₁₁ to L₆₁₃ may each be the same as described herein with respect to L₆₀₁,
xe611 to xe613 may each be the same as described herein with respect to xe1,
R₆₁₁ to R₆₁₃ may each be the same as described herein with respect to R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group unsubstituted or substituted with at least one R₁₀ₐ, or a C₁-C₆₀ heterocyclic group unsubstituted or substituted with at least one R₁₀ₐ.

For example, xe1 and xe611 to xe613 in Formulae 601 and 601-1 may each independently be 0, 1, or 2.

The electron transport region may include one of Compounds ET1 to ET45, 2,9-dimethyl-4,7-diphenyl-1,10-phenanthroline (BCP), 4,7-diphenyl-1,10-phenanthroline (Bphen), Alq₃, BAlq, TAZ, NTAZ, or any combination thereof:

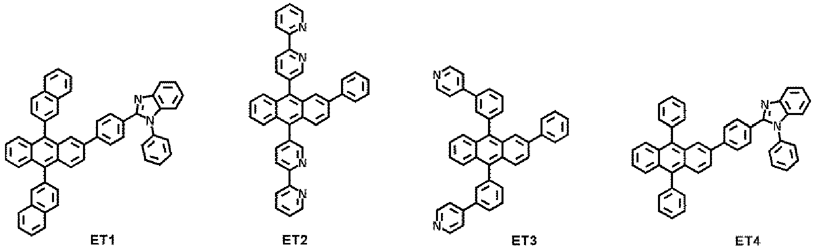

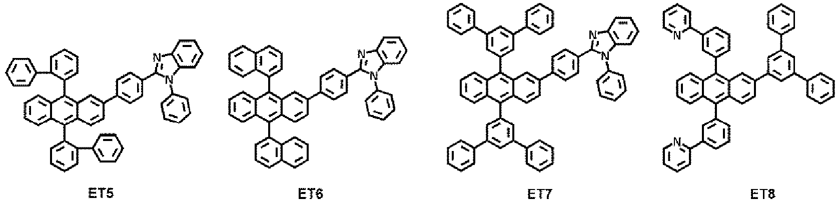

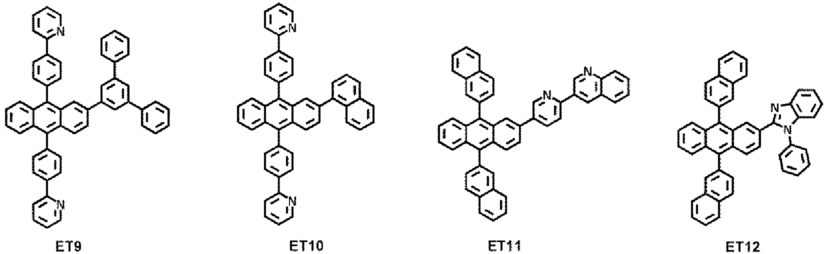

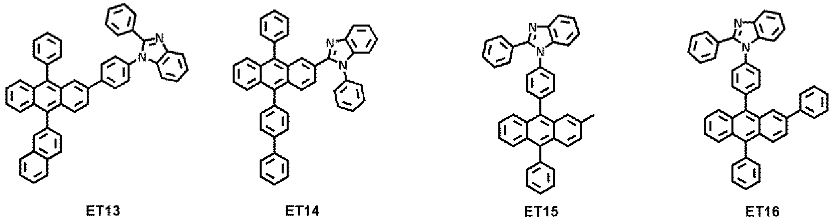

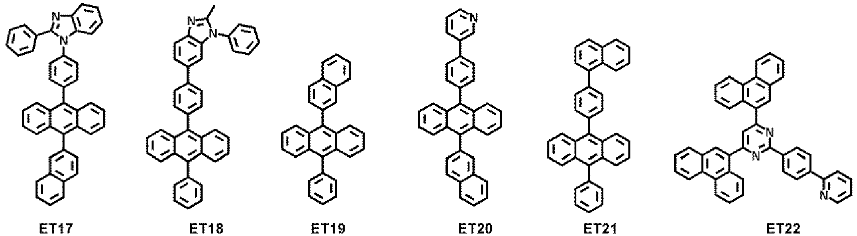

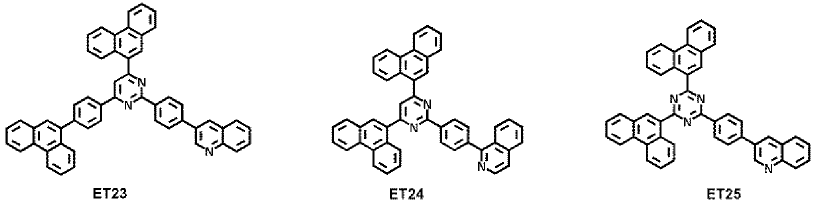

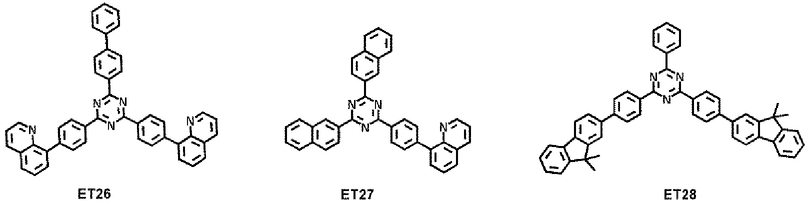

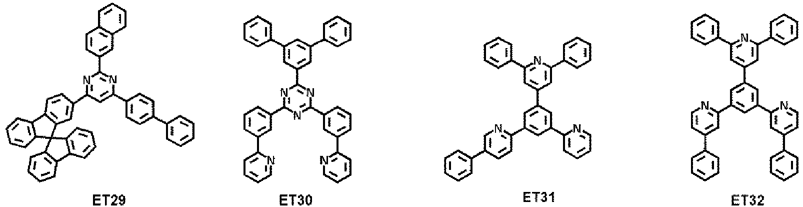

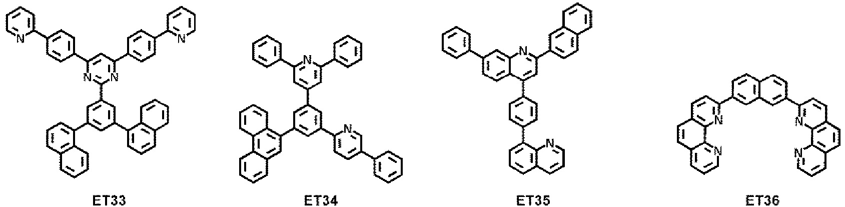

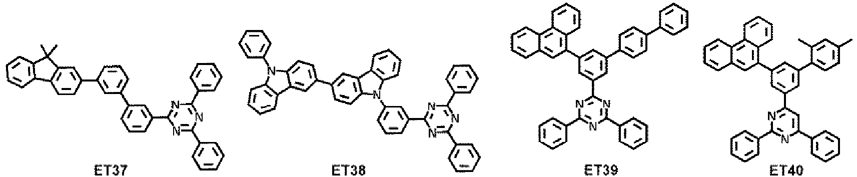

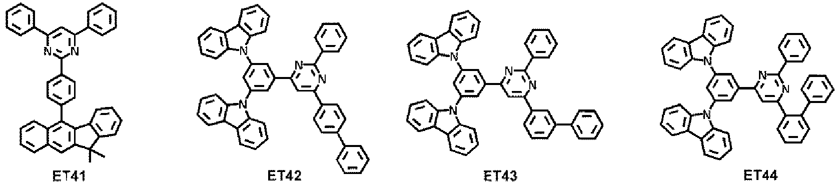

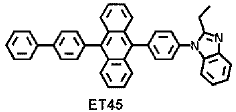

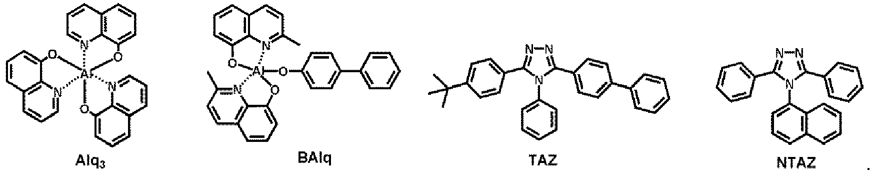

A thickness of the electron transport region may be from about 100 Å to about 5,000 Å, for example, about 160 Å to about 4,000 Å. When the electron transport region includes the hole blocking layer, the electron transport layer, or any combination thereof, a thickness of the hole blocking layer may be from about 20 Å to about 1,000 Å, for example, about 30 Å to about 300 Å, and the thickness of the electron transport layer may be from about 100 Å to about 1,000 Å, for example, about 150 Å to about 500 Å. When the thicknesses of the hole blocking layer and/or the electron transport layer are within these ranges, satisfactory electron transporting characteristics may be obtained without a substantial increase in driving voltage.

The electron transport region (for example, the electron transport layer in the electron transport region) may further include, in addition to the materials described above, a metal-containing material.

The metal-containing material may include an alkali metal complex, an alkaline earth metal complex, or any combination thereof. The metal ion of an alkali metal complex may be a Li ion, a Na ion, a K ion, a Rb ion, or a Cs ion, and the metal ion of an alkaline earth metal complex may be a Be ion, a Mg ion, a Ca ion, a Sr ion, or a Ba ion. A ligand coordinated with the metal ion of the alkali metal complex or the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a Li complex. The Li complex may include, for example, Compound ET-D1 (Liq) or ET-D2:

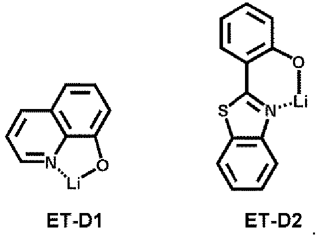

The electron transport region may include an electron injection layer that facilitates the injection of electrons from the second electrode 150. The electron injection layer may directly contact the second electrode 150.

The electron injection layer may have: i) a single-layered structure consisting of a single layer consisting of a single material, ii) a single-layered structure consisting of a single layer consisting of a plurality of different materials, or iii) a multi-layered structure including a plurality of layers including different materials.

The electron injection layer may include an alkali metal, alkaline earth metal, a rare earth metal, an alkali metal-containing compound, alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may be oxides, halides (for example, fluorides, chlorides, bromides, or iodides), or tellurides of the alkali metal, the alkaline earth metal, and the rare earth metal, or any combination thereof.

The alkali metal-containing compound may include alkali metal oxides, such as Li₂O, Cs₂O, or K₂O; alkali metal halides, such as LiF, NaF, CsF, KF, Lil, Nal, Csl, or KI; or any combination thereof. The alkaline earth metal-containing compound may include an alkaline earth metal oxide, such as BaO, SrO, CaO, BaₓSr₁₋ₓO (wherein x is a real number satisfying the condition of 0<x<1), BaₓCa₁₋ₓO (wherein x is a real number satisfying the condition of 0<x<1), or the like. The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In one or more embodiments, the rare earth metal-containing compound may include lanthanide metal telluride. Examples of the lanthanide metal telluride are LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, and Lu₂Te₃.

The alkali metal complex, the alkaline earth-metal complex, and the rare earth metal complex may include i) one of ions of the alkali metal, the alkaline earth metal, and the rare earth metal and ii), as a ligand bonded to the metal ion, for example, a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenyl benzimidazole, a hydroxyphenylbenzothiazole, a bipyridine, a phenanthroline, a cyclopentadiene, or any combination thereof.

The electron injection layer may consist of an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In one or more embodiments, the electron injection layer may further include an organic material (for example, a compound represented by Formula 601).

In one or more embodiments, the electron injection layer may consist of: i) an alkali metal-containing compound (for example, an alkali metal halide); or ii) a) an alkali metal-containing compound (for example, an alkali metal halide), and b) an alkali metal, an alkaline earth metal, a rare earth metal, or any combination thereof. For example, the electron injection layer may be a KI:Yb co-deposited layer, an Rbl:Yb co-deposited layer, or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 1 Å to about 100 Å, for example, about 3 Å to about 90 Å. When the thickness of the electron injection layer is within the ranges described above, satisfactory electron injection characteristics may be obtained without a substantial increase in driving voltage.

The second electrode 150 may be located on the interlayer 130 having a structure as described above. The second electrode 150 may be a cathode, which is an electron injection electrode, and as the material for the second electrode 150, a metal, an alloy, an electrically conductive compound, or any combination thereof, each having a low-work function, may be used.

The second electrode 150 may include lithium (Li), silver (Ag), magnesium (Mg), aluminium (Al), aluminium-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a semi-transmissive electrode, or a reflective electrode.

The second electrode 150 may have a single-layered structure or a multi-layered structure including a plurality of layers.

### Capping Layer

A first capping layer may be located outside the first electrode 110, and/or a second capping layer may be located outside the second electrode 150. In particular, the light-emitting device 10 may have a structure in which the first capping layer, the first electrode 110, the interlayer 130, and the second electrode 150 are sequentially stacked in the stated order, a structure in which the first electrode 110, the interlayer 130, the second electrode 150, and the second capping layer are sequentially stacked in the stated order, or a structure in which the first capping layer, the first electrode 110, the interlayer 130, the second electrode 150, and the second capping layer are sequentially stacked in the stated order.

Light generated in an emission layer of the interlayer 130 of the light-emitting device 10 may be extracted toward the outside through the first electrode 110 which is a semi-transmissive electrode or a transmissive electrode, and the first capping layer. Light generated in an emission layer of the interlayer 130 of the light-emitting device 10 may be extracted toward the outside through the second electrode 150 which is a semi-transmissive electrode or a transmissive electrode, and the second capping layer.

The first capping layer and the second capping layer may increase external emission efficiency according to the principle of constructive interference. Accordingly, the light extraction efficiency of the light-emitting device 10 is increased, so that the luminescence efficiency of the light-emitting device 10 may be improved.

Each of the first capping layer and the second capping layer may include a material having a refractive index of 1.6 or more (at 589 nm).

The first capping layer and the second capping layer may each independently be an organic capping layer including an organic material, an inorganic capping layer including an inorganic material, or an organic-inorganic composite capping layer including an organic material and an inorganic material.

At least one of the first capping layer and the second capping layer may each independently include carbocyclic compounds, heterocyclic compounds, amine group-containing compounds, porphine derivatives, phthalocyanine derivatives, naphthalocyanine derivatives, alkali metal complexes, alkaline earth metal complexes, or any combination thereof. Optionally, the carbocyclic compound, the heterocyclic compound, and the amine group-containing compound may be substituted with a substituent including O, N, S, Se, Si, F, CI, Br, I, or any combination thereof. In one or more embodiments, at least one of the first capping layer and the second capping layer may each independently include an amine group-containing compound.

For example, at least one of the first capping layer and the second capping layer may each independently include a compound represented by Formula 201, a compound represented by Formula 202, or any combination thereof.

In one or more embodiments, at least one of the first capping layer and the second capping layer may each independently include one of Compounds HT28 to HT33, one of Compounds CP1 to CP6, β-NPB, or any combination thereof:

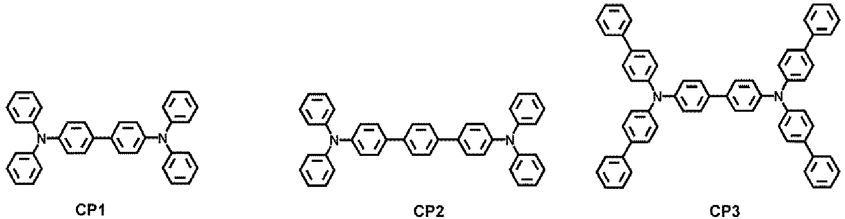

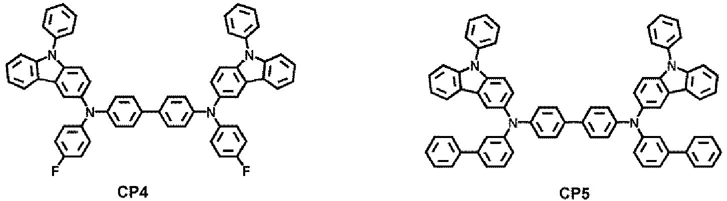

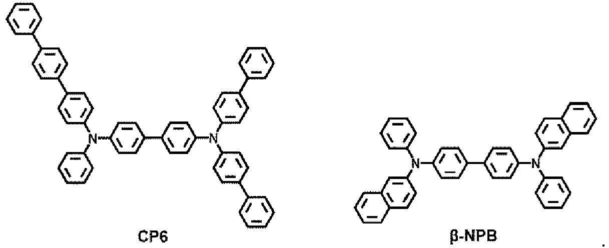

### Electronic apparatus

The light-emitting device may be included in various electronic apparatuses. For example, the electronic apparatus including the light-emitting device may be a light-emitting apparatus, an authentication apparatus, or the like.

The electronic apparatus (for example, a light-emitting apparatus) may further include, in addition to the light-emitting device, i) a color filter, ii) a color conversion layer, or iii) a color filter and a color conversion layer. The color filter and/or the color conversion layer may be located in at least one direction in which light emitted from the light-emitting device travels. For example, the light emitted from the light-emitting device may be blue light or white light. For details on the light-emitting device, related description provided above may be referred to.

The electronic apparatus may include a first substrate. The first substrate may include a plurality of subpixel areas, the color filter may include a plurality of color filter areas respectively corresponding to the plurality of subpixel areas, and the color conversion layer may include a plurality of color conversion areas respectively corresponding to the plurality of subpixel areas.

A pixel defining layer may be located among the plurality of subpixel areas to define each of the plurality of subpixel areas.

The color filter may further include a plurality of color filter areas and light-shielding patterns located among the plurality of color filter areas, and the color conversion layer may further include a plurality of color conversion areas and light-shielding patterns located among the plurality of color conversion areas.

The plurality of color filter areas (or the plurality of color conversion areas) may include a first area emitting first color light, a second area emitting second color light, and/or a third area emitting third color light, wherein the first color light, the second color light, and/or the third color light may have different maximum emission wavelengths from one another. For example, the first color light may be red light, the second color light may be green light, and the third color light may be blue light. For example, the plurality of color filter areas (or the plurality of color conversion areas) may include quantum dots. In particular, the first area may include a red quantum dot, the second area may include a green quantum dot, and the third area may not include a quantum dot. For details on the quantum dot, related descriptions provided herein may be referred to. The first area, the second area, and/or the third area may each include a scatterer.

The areas including quantum dots may be formed using a composition including quantum dots coordinated with the compound as described herein.

For example, the light-emitting device may emit a first light, the first area may absorb the first light to emit first-first color light, the second area may absorb the first light to emit second-first color light, and the third area may absorb the first light to emit third-first color light. In this regard, the first-first color light, the second-first color light, and the third-first color light may have different maximum emission wavelengths. In particular, the first light may be blue light, the first-first color light may be red light, the second-first color light may be green light, and the third-first color light may be blue light.

The electronic apparatus may further include a thin-film transistor, in addition to the light-emitting device as described above. The thin-film transistor may include a source electrode, a drain electrode, and an activation layer, wherein any one of the source electrode and the drain electrode may be electrically connected to any one of the first electrode and the second electrode of the light-emitting device.

The thin-film transistor may further include a gate electrode, a gate insulating film, or the like.

The activation layer may include crystalline silicon, amorphous silicon, an organic semiconductor, an oxide semiconductor, or the like.

The electronic apparatus may further include a sealing portion for sealing the light-emitting device. The sealing portion may be located between the color filter and/or the color conversion layer and the light-emitting device. The sealing portion allows light from the light-emitting device to be extracted to the outside, and simultaneously prevents ambient air and moisture from penetrating into the light-emitting device. The sealing portion may be a sealing substrate including a transparent glass substrate or a plastic substrate. The sealing portion may be a thin-film encapsulation layer including at least one layer of an organic layer and an inorganic layer. When the sealing portion is a thin film encapsulation layer, the electronic apparatus may be flexible.

Various functional layers may be additionally located on the sealing portion, in addition to the color filter and/or the color conversion layer, according to the use of the electronic apparatus. Examples of the functional layers may include a touch screen layer, a polarizing layer, and the like. The touch screen layer may be a pressure-sensitive touch screen layer, a capacitive touch screen layer, or an infrared touch screen layer. The authentication apparatus may be, for example, a biometric authentication apparatus that authenticates an individual by using biometric information of a living body (for example, fingertips, pupils, etc.).

The authentication apparatus may further include, in addition to the light-emitting device as described above, a biometric information collector.

The electronic apparatus may be applied to various displays, light sources, lighting apparatus, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various measuring instruments, meters (for example, meters for a vehicle, an aircraft, and a vessel), projectors, and the like.

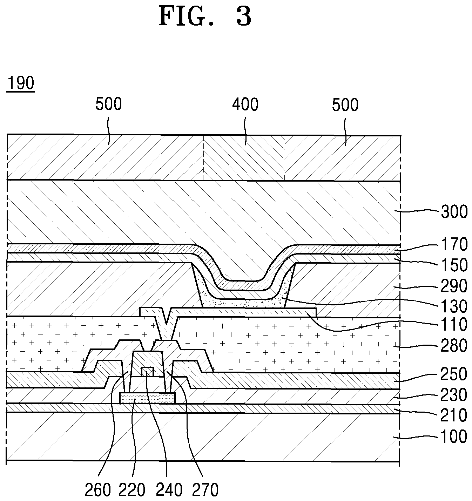

### Description of Figures 2 and 3

FIG. 2 is a cross-sectional view of an electronic apparatus 180 according to an embodiment of the disclosure. The electronic apparatus 180 of FIG. 2 includes a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be located on the substrate 100. The buffer layer 210 may prevent penetration of impurities through the substrate 100 and may provide a more flat or planar surface that a surface of the substrate 100.

A TFT may be located on the buffer layer 210. The TFT may include an activation layer 220, a gate electrode 240, a source electrode 260, and a drain electrode 270.

The activation layer 220 may include an inorganic semiconductor such as silicon or polysilicon, an organic semiconductor, or an oxide semiconductor, and may include a source region, a drain region, and a channel region. A gate insulating film 230 for insulating the activation layer 220 from the gate electrode 240 may be located on the activation layer 220, and the gate electrode 240 may be located on the gate insulating film 230.

An interlayer insulating film 250 may be located on the gate electrode 240. The interlayer insulating film 250 may be located between the gate electrode 240 and the source electrode 260 and between the gate electrode 240 and the drain electrode 270, to insulate from one another. The source electrode 260 and the drain electrode 270 may be located on the interlayer insulating film 250. The interlayer insulating film 250 and the gate insulating film 230 may be formed to expose the source region and the drain region of the activation layer 220, and the source electrode 260 and the drain electrode 270 may be located in contact with the exposed portions of the source region and the drain region of the activation layer 220.

The TFT is electrically connected to a light-emitting device to drive the light-emitting device and is covered and protected by a passivation layer 280. The passivation layer 280 may include an inorganic insulating film, an organic insulating film, or any combination thereof. A light-emitting device is provided on the passivation layer 280. The light-emitting device may include a first electrode 110, an interlayer 130, and a second electrode 150.

The first electrode 110 may be located on the passivation layer 280. The passivation layer 280 may be located to expose a portion of the drain electrode 270, not fully covering the drain electrode 270, and the first electrode 110 may be located to be connected to the exposed portion of the drain electrode 270.

A pixel defining layer 290 including an insulating material may be located on the first electrode 110. The pixel defining layer 290 may expose a certain region of the first electrode 110, and an interlayer 130 may be formed in the exposed region of the first electrode 110. The pixel defining layer 290 may be a polyimide or polyacrylic organic film. Although not shown in FIG. 2, at least some layers of the interlayer 130 may extend beyond the upper portion of the pixel defining layer 290 to be located in the form of a common layer.

The second electrode 150 may be located on the interlayer 130, and a capping layer 170 may be additionally formed on the second electrode 150. The capping layer 170 may be formed to cover the second electrode 150.

The encapsulation portion 300 may be located on the capping layer 170. The encapsulation portion 300 may be located on a light-emitting device to protect the light-emitting device from moisture or oxygen. The encapsulation portion 300 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic-based resin (for example, polymethyl methacrylate, polyacrylic acid, or the like), an epoxy-based resin (for example, aliphatic glycidyl ether (AGE), or the like), or any combination thereof; or any combination of the inorganic films and the organic films.

FIG. 3 is a cross-sectional view of an electronic apparatus 190 according to an embodiment of the disclosure. The electronic apparatus 190 of FIG. 3 is substantially the same as the electronic apparatus 180 of FIG. 2, except that a light-shielding pattern 500 and a functional region 400 are additionally arranged on the encapsulation portion 300. The functional region 400 may be i) a color filter area, ii) a color conversion area, or iii) a combination of the color filter area and the color conversion area. In one or more embodiments, the light-emitting device included in the electronic apparatus of FIG. 3 may be a tandem light-emitting device.

### Manufacturing method

Respective layers included in the hole transport region, the emission layer, and respective layers included in the electron transport region may be formed in a certain region by using one or more suitable methods selected from vacuum deposition, spin coating, casting, Langmuir-Blodgett (LB) deposition, ink-jet printing, laser-printing, laser-induced thermal imaging (LITI), and the like.

The color filter area, the color conversion area, etc. may be formed in a certain region using a spin coating method, a casting method, an ink jet printing method, or the like.

When respective layers included in the hole transport region, an emission layer, and respective layers included in the electron transport region are formed by vacuum deposition, the deposition may be performed at a deposition temperature of about 100 °C to about 500 °C, a vacuum degree of about 10⁻⁸ torr to about 10⁻³ torr, and a deposition speed of about 0.01 angstroms per second (Å/sec) to about 100 Å/sec, depending on a material to be included in a layer to be formed and the structure of a layer to be formed.

When respective layers included in the hole transport region, an emission layer, and respective layers included in the electron transport region are formed by spin coating, the spin coating may be performed at a coating speed of about 2,000 rpm to about 5,000 rpm and at a heat treatment temperature of about 80 °C to about 200 °C by taking into account a material to be included in a layer to be formed and the structure of a layer to be formed.

The composition according to an embodiment may be used in a solution process such as a spin coating method or an inkjet printing method.

### Definition of terms

The term "C₃-C₆₀ carbocyclic group" as used herein refers to a cyclic group consisting of carbon atoms only as a ring-forming atom and having three to sixty carbon atoms, and the term "C₁-C₆₀ heterocyclic group" as used herein refers to a cyclic group that has one to sixty carbon atoms and further has, in addition to carbon atoms, a heteroatom as a ring-forming atom. The C₃-C₆₀ carbocyclic group and the C₁-C₆₀ heterocyclic group may each be a monocyclic group consisting of one ring or a polycyclic group in which two or more rings are condensed with each other. For example, the C₁-C₆₀ heterocyclic group has 3 to 61 ring-forming atoms.

The "cyclic group" as used herein may include the C₃-C₆₀ carbocyclic group, and the C₁-C₆₀ heterocyclic group.

The term "π electron-rich C₃-C₆₀ cyclic group" as used herein refers to a cyclic group that has three to sixty carbon atoms and does not include *-N=*' as a ring-forming moiety, and the term "π electron-deficient nitrogen-containing C₁-C₆₀ cyclic group" as used herein refers to a heterocyclic group that has one to sixty carbon atoms and includes *-N=*' as a ring-forming moiety.

For example,
the C₃-C₆₀ carbocyclic group may be i) a group T1 or ii) a condensed cyclic group in which two or more groups T1 are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
the C₁-C₆₀ heterocyclic group may be i) a group T2, ii) a condensed cyclic group in which two or more groups T2 are condensed with each other, or iii) a condensed cyclic group in which at least one group T2 and at least one group T1 are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.),
the π electron-rich C₃-C₆₀ cyclic group may be i) a group T1, ii) a condensed cyclic group in which two or more groups T1 are condensed with each other, iii) a group T3, iv) a condensed cyclic group in which two or more groups T3 are condensed with each other, or v) a condensed cyclic group in which at least one group T3 and at least one group T1 are condensed with each other (for example, the C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, etc.),
the π electron-deficient nitrogen-containing C₁-C₆₀ cyclic group may be i) a group T4, ii) a condensed cyclic group in which two or more groups T4 are condensed with each other, iii) a condensed cyclic group in which at least one group T4 and at least one group T1 are condensed with each other, iv) a condensed cyclic group in which at least one group T4 and at least one group T3 are condensed with each other, or v) a condensed cyclic group in which at least one group T4, at least one group T1, and at least one group T3 are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.),
the group T1 may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or a bicyclo[2.2.1]heptane) group, a norbornene group, a bicyclo[1.1.1]pentane group, a bicyclo[2.1.1]hexane group, a bicyclo[2.2.2]octane group, or a benzene group,
the group T2 may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
the group T3 may be a furan group, a thiophene group, a 1H-pyrrole group, a silole group, or a borole group, and
the group T4 may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.
The terms "the cyclic group, the C₃-C₆₀ carbocyclic group, the C₁-C₆₀ heterocyclic group, the π electron-rich C₃-C₆₀ cyclic group, or the π electron-deficient nitrogen-containing C₁-C₆₀ cyclic group" as used herein refer to a group condensed to any cyclic group, a monovalent group, or a polyvalent group (for example, a divalent group, a trivalent group, a tetravalent group, etc.) according to the structure of a formula for which the corresponding term is used. For example, the "benzene group" may be a benzo group, a phenyl group, a phenylene group, or the like, which may be easily understood by one of ordinary skill in the art according to the structure of a formula including the "benzene group."
Examples of the monovalent C₃-C₆₀ carbocyclic group and the monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, and examples of the divalent C₃-C₆₀ carbocyclic group and the divalent C₁-C₆₀ heterocyclic group may include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group.

The term "C₁-C₆₀ alkyl group" as used herein refers to a linear or branched aliphatic hydrocarbon monovalent group that has one to sixty carbon atoms, and specific examples thereof are a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group. The term "C₁-C₆₀ alkylene group" as used herein refers to a divalent group having the same structure as the C₁-C₆₀ alkyl group.

The term "C₂-C₆₀ alkenyl group" as used herein refers to a monovalent hydrocarbon group having at least one carbon-carbon double bond in the middle or at the terminus of the C₂-C₆₀ alkyl group, and examples thereof are an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a monovalent hydrocarbon group having at least one carbon-carbon triple bond in the middle or at the terminus of the C₂-C₆₀ alkyl group, and examples thereof include an ethynyl group, a propynyl group, and the like. The term "C₂-C₆₀ alkynylene group" as used herein refers to a divalent group having the same structure as the C₂-C₆₀ alkynyl group.

The term "C₁-C₆₀ alkoxy group" as used herein refers to a monovalent group represented by -OA₁₀₁ (wherein A₁₀₁ is the C₁-C₆₀ alkyl group), and examples thereof include a methoxy group, an ethoxy group, and an isopropyloxy group.

The term "C₃-C₁₀ cycloalkyl group" as used herein refers to a monovalent saturated hydrocarbon cyclic group having 3 to 10 carbon atoms, and examples thereof are a cyclopropyl group, a cyclobutyl group, a cyclopentyl group, a cyclohexyl group, a cycloheptyl group, a cyclooctyl group, an adamantanyl group, a norbornyl group (or bicyclo[2.2.1]heptyl group), a bicyclo[1.1.1]pentyl group, a bicyclo[2.1.1]hexyl group, and a bicyclo[2.2.2]octyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent cyclic group of 1 to 10 carbon atoms, further including, in addition to carbon atoms, at least one heteroatom, as ring-forming atoms, and specific examples are a 1,2,3,4-oxatriazolidinyl group, a tetrahydrofuranyl group, and a tetrahydrothienyl group. The term "C₁-C₁₀ heterocycloalkylene group" as used herein refers to a divalent group having the same structure as the C₁-C₁₀ heterocycloalkyl group.

The term C₃-C₁₀ cycloalkenyl group used herein refers to a monovalent cyclic group that has three to ten carbon atoms and at least one carbon-carbon double bond in the ring thereof and no aromaticity, and specific examples thereof are a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent cyclic group of 1 to 10 carbon atoms, further including, in addition to carbon atoms, at least one heteroatom, as ring-forming atoms, and having at least one double bond in the cyclic structure thereof. Examples of the C₁-C₁₀ heterocycloalkenyl group include a 4,5-dihydro-1,2,3,4-oxatriazolyl group, a 2,3-dihydrofuranyl group, and a 2,3-dihydrothienyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system of 6 to 60 ring carbon atoms, and the term "C₆-C₆₀ arylene group" as used herein refers to a divalent group having a carbocyclic aromatic system of 6 to 60 carbon ring atoms. Examples of the C₆-C₆₀ aryl group are a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group. When the C₆-C₆₀ aryl group and the C₆-C₆₀ arylene group each include two or more rings, the rings may be condensed with each other.

The term "C₁-C₆₀ heteroaryl group" as used herein refers to a monovalent group having a heterocyclic aromatic system of 1 to 60 carbon ring atoms, further including, in addition to carbon atoms, at least one heteroatom, as ring-forming atoms. The term "C₁-C₆₀ heteroarylene group" as used herein refers to a divalent group having a heterocyclic aromatic system of 1 to 60 carbon ring atoms, further including, in addition to carbon atoms, at least one heteroatom, as ring-forming atoms. Examples of the C₁-C₆₀ heteroaryl group are a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group. When the C₁-C₆₀ heteroaryl group and the C₁-C₆₀ heteroarylene group each include two or more rings, the rings may be condensed with each other.

The term "monovalent non-aromatic condensed polycyclic group" as used herein refers to a monovalent group (for example, having 8 to 60 carbon ring atoms) having two or more rings condensed to each other, only carbon atoms as ring-forming atoms, and no aromaticity in its entire molecular structure. Examples of the monovalent non-aromatic condensed polycyclic group are an indenyl group, a fluorenyl group, a spiro-bifluorenyl group, a benzofluorenyl group, an indenophenanthrenyl group, and an indeno anthracenyl group. The term "divalent non-aromatic condensed polycyclic group" as used herein refers to a divalent group having the same structure as the monovalent non-aromatic condensed polycyclic group described above.

The term "monovalent non-aromatic condensed heteropolycyclic group" as used herein refers to a monovalent group (for example, having 1 to 60 carbon atoms) having two or more rings condensed to each other, further including, in addition to carbon atoms, at least one heteroatom, as ring-forming atoms, and having non-aromaticity in its entire molecular structure. Examples of the monovalent non-aromatic condensed heteropolycyclic group include a pyrrolyl group, a thienyl group, a furanyl group, an indolyl group, a benzoindolyl group, a naphtho indolyl group, an isoindolyl group, a benzoisoindolyl group, a naphthoisoindolyl group, a benzosilolyl group, a benzothienyl group, a benzofuranyl group, a carbazolyl group, a dibenzosilolyl group, a dibenzothienyl group, a dibenzofuranyl group, an azacarbazolyl group, an azafluorenyl group, an azadibenzosilolyl group, an azadibenzothienyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indenocarbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothienyl group, a benzonaphthosilolyl group, a benzofurodibenzofuranyl group, a benzofurodibenzothienyl group, and a benzothienodibenzothienyl group. The term "divalent non-aromatic condensed heteropolycyclic group" as used herein refers to a divalent group having the same structure as the monovalent non-aromatic condensed heteropolycyclic group described above.
The term "C₆-C₆₀ aryloxy group" as used herein refers to -OA₁₀₂ (wherein A₁₀₂ is the C₆-C₆₀ aryl group), and the term "C₆-C₆₀ arylthio group" as used herein refers to -SA₁₀₃ (wherein A₁₀₃ is the C₆-C₆₀ aryl group).

The term "C₇-C₆₀ aryl alkyl group" used herein refers to -A₁₀₄A₁₀₅ (where A₁₀₄ may be a C₁-C₅₄ alkylene group, and A₁₀₅ may be a C₆-C₅₉ aryl group), and the term C₂-C₆₀ heteroaryl alkyl group" used herein refers to -A₁₀₆A₁₀₇ (where A₁₀₆ may be a C₁-C₅₉ alkylene group, and A₁₀₇ may be a C₁-C₅₉ heteroaryl group).

The term "R₁₀ₐ" as used herein refers to:
deuterium, -F, -Cl, -Br, -I, a hydroxyl group, a cyano group, or a nitro group,
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), -P(=O)(Q₁₁)(Q₁₂), or any combination thereof,
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂),
Q₁, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group, a C₁-C₆₀ heterocyclic group, a C₇-C₆₀ arylalkyl group, or a C₂-C₆₀ heteroaryl alkyl group, each substituted with deuterium, -F, a cyano group, a C₁-C₆₀ alkyl group, a C₁-C₆₀ alkoxy group, a phenyl group, a biphenyl group, or any combination thereof.

The term "heteroatom" as used herein refers to any atom other than a carbon atom and a hydrogen atom. Examples of the heteroatom are O, S, N, P, Si, B, Ge, Se, and any combinations thereof.

The term "the transition metal" used herein includes hafnium (Hf), tantalum (Ta), tungsten (W), rhenium (Re), osmium (Os), iridium (Ir), platinum (Pt), gold (Au), etc.

"Ph" as used herein refers to a phenyl group, "Me" as used herein refers to a methyl group, "Et" as used herein refers to an ethyl group, "tert-Bu" or "Bu^{t}" as used herein refers to a tert-butyl group, and "OMe" as used herein refers to a methoxy group.

The term "biphenyl group" as used herein refers to "a phenyl group substituted with a phenyl group." In other words, the "biphenyl group" is a substituted phenyl group having a C₆-C₆₀ aryl group as a substituent.

The term "terphenyl group" as used herein refers to "a phenyl group substituted with a biphenyl group". In other words, the "terphenyl group" is a substituted phenyl group having, as a substituent, a C₆-C₆₀ aryl group substituted with a C₆-C₆₀ aryl group.

The maximum number of carbon atoms in this substituent definition section is an example only. In an embodiment, the maximum carbon number of 60 in the C₁-C₆₀ alkyl group is an example, and the definition of the alkyl group is equally applied to a C₁-C₂₀ alkyl group. The same applies to other cases.

* and *' as used herein, unless defined otherwise, each refer to a binding site to a neighboring atom in a corresponding formula.

Hereinafter, a compound and light-emitting device according to embodiments will be described in detail with reference to Examples.

### Ligand Synthesis

### Example 1

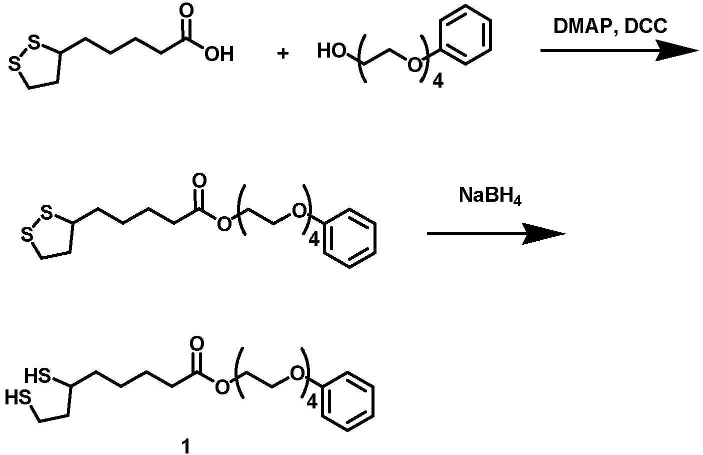

A mixed solution of 232 grams (g) of tetraethylene glycol monophenyl ether, 24.8 g of thioctic acid (alpha-lipoic acid), 4.4 g of 4-(N,N-dimethylamino)pyridine (DMAP), and 1,200 milliliters (ml) of dichloromethane was put into a flask in an ice bath (i.e., kept cool with ice water), and a nitrogen atmosphere (gas) was added to the flask for 30 minutes, and a nitrogen atmosphere was maintained until the reaction was completed.

While stirring the reaction mixture at or near 0 °C a solution of 27.2 g of N,N'-dicyclohexylcarbodiimide (DCC) dissolved in 80 ml of dichloromethane was slowly added dropwise to the flask over 40 minutes, and the reaction was stirred at or near 0 °C for 1 hour. The reaction temperature was slowly raised to room temperature and stirred for an additional 18 hours.

The reaction mixture was mixed with 1,200 ml of saturated sodium bicarbonate aqueous solution, followed by an addition of 400 ml of ethyl acetate to extract an intermediate. The organic layer was separated and the solvent was evaporated and the obtained product was dried. The intermediate was purified by column chromatography.

16 g of the obtained intermediate was dissolved in 200 ml of an ethanol:water 1:4 (vol/vol) mixture and stirred. To proceed with the reduction reaction, 1.7 g of NaBH₄ was added to the mixture and stirred for 60 minutes in a nitrogen atmosphere, 400 ml of brine was added thereto, and was extracted three times with chloroform.

The solvent was evaporated and dried to obtain Compound 1.

### Example 2

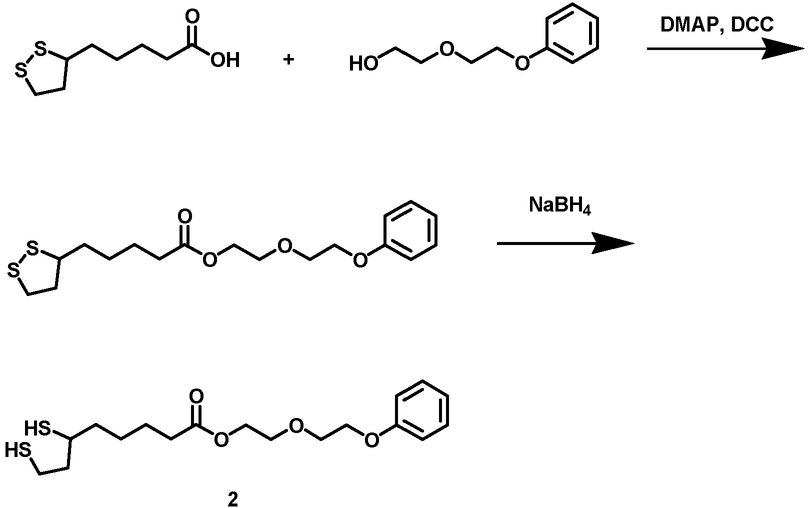

Compound 2 was obtained in substantially the same manner as Example 1 was obtained except that 170 g of triethylene glycol monophenyl ether was used instead of tetraethylene glycol monophenyl ether.

### Example 3

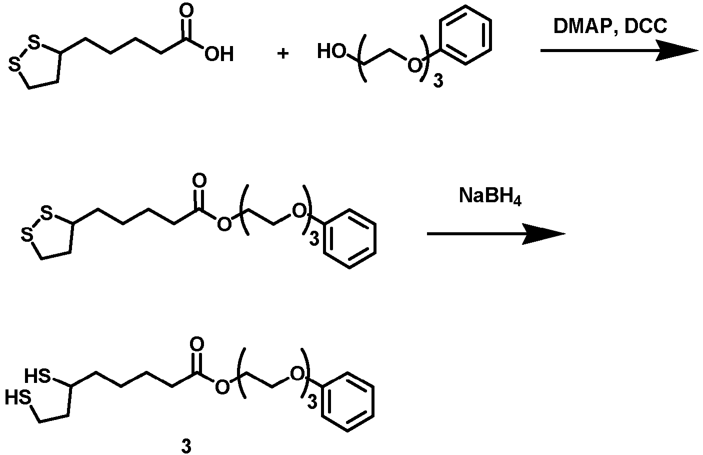

Compound 3 was obtained in substantially the same manner as Example 1 was obtained except that 200 g of triethylene glycol monophenyl ether was used instead of tetraethylene glycol monophenyl ether.

### Example 4

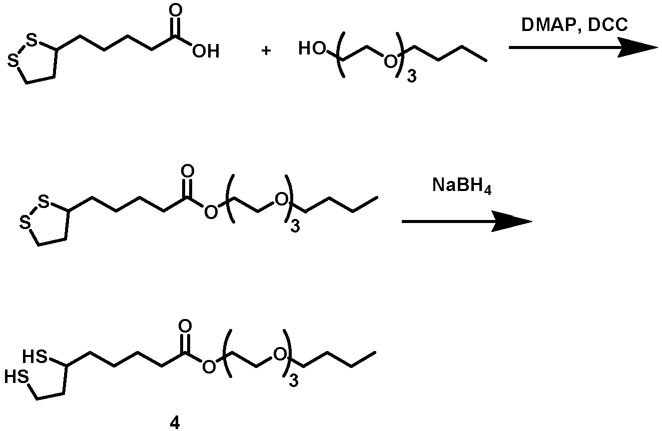

Compound 4 was obtained in substantially the same manner as Example 1 was obtained except that 200 g of triethylene glycol monobutyl ether was used instead of tetraethylene glycol monophenyl ether.

### Example 5

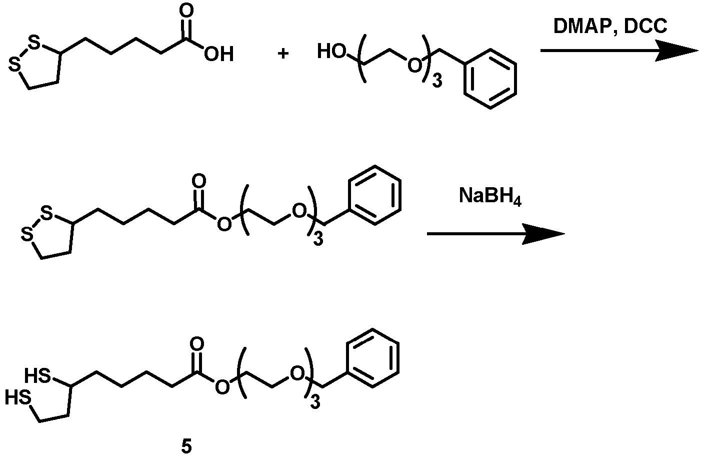

Compound 5 was obtained in substantially the same manner as Example 1 was obtained except that 200 g of triethylene glycol monobenzyl ether was used instead of tetraethylene glycol monophenyl ether.

The compositions were manufacturing using the quantum dots and ligands of Table 1.

**Table 1**

| | Quantum dot (10 nm) | Ligand |
|---|---|---|
| Comparative Example 1 | ZnS shell and Group II-V core | Compound 101 |
| Comparative Example 2 | ZnS shell and Group II-V core | Compound 102 |
| Comparative Example 3 | ZnS shell and Group III-V core | Compound 103 |
| Comparative Example 4 | ZnS shell and Group III-V core | Compound 104 |
| Comparative Example 5 | ZnS shell and Group II-V core | Compound 105 |
| Example 6 | ZnS shell and Group II-V core | Compound 1 |
| Example 7 | ZnS shell and Group II-V core | Compound 2 |
| Example 8 | ZnS shell and Group III-V core | Compound 3 |
| Example 9 | ZnS shell and Group III-V core | Compound 4 |
| Example 10 | ZnS shell and Group II-V core | Compound 5 |

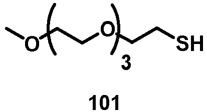

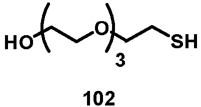

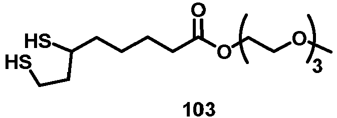

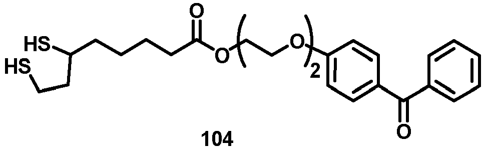

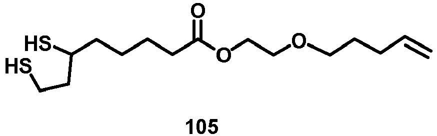

### Comparative Example 1

1 g of the quantum dots of Table 1 were added to chloroform in an amount of 25 wt%, followed by stirring at room temperature for 1 hour. After adding 0.238 g of Compound 101, the mixture was stirred at 70 °C for 2 hours.
Hexane is added to the quantum dot solution at a volume ratio of about 10 fold with respect to the reaction solution, and the quantum dots are obtained (separated) by centrifugation (9500 rpm / 3 min) and vacuum dried to obtain a quantum dot(s) in which a native ligand [oleic acid] of the quantum dot was substituted for Compound 101.

0.375 g of the quantum dot and 0.526 g of the crosslinking monomer 1,6-hexanediol diacrylate, the mixture was added to a reaction flask and shaken for 12 hours. Afterwards, 0.08 g of TiO₂ and 0.01 g of a photoinitiator, diphenyl(2,4,6-trimethylbenzoyl)phosphine oxide, were added, and the mixture was shaken for 3 hours to form a resin composition including the quantum dots.

### Comparative Example 2

A composition was manufactured in substantially the same manner as in Comparative Example 1 except that 0.374 g of Compound 102 was used instead of Compound 101.

### Comparative Example 3

A composition was manufactured in substantially the same manner as in Comparative Example 1 except that 0.377 g of Compound 103 was used instead of Compound 101.

### Comparative Example 4

A composition was manufactured in substantially the same manner as in Comparative Example 1 except that 0.508 g of Compound 104 was used instead of Compound 101.

### Comparative Example 5

A composition was manufactured in substantially the same manner as in Comparative Example 1 except that 0.339 g of Compound 105 was used instead of Compound 101.

### Example 6

A composition was manufactured in substantially the same manner as in Comparative Example 1 except that 0.489 g of Compound 1 was used instead of Compound 101.

### Example 7

A composition was manufactured in substantially the same manner as in Comparative Example 1 except that 0.396 g of Compound 2 was used instead of Compound 101.

### Example 8

A composition was manufactured in substantially the same manner as in Comparative Example 1 except that 0.442 g of Compound 3 was used instead of Compound 101.

### Example 9

A composition was manufactured in substantially the same manner as in Comparative Example 1 except that 0.421 g of Compound 4 was used instead of Compound 101.

### Example 10

A composition was manufactured in substantially the same manner as in Comparative Example 1 except that 0.457 g of Compound 5 was used instead of Compound 101.

The initial viscosity (at 25 °C) of the comparative examples and examples was about 25 centipoise (cP) to about 30 cP.

### Evaluation on viscosity change

The initial viscosity of the comparative examples and the examples was measured, and then, after 30 days, the viscosity was measured to evaluate the change in viscosity over time. The results thereof are shown in Table 2. A Brookfield viscometer DV3 was used as a viscometer.

**Table 2**

| | Change in viscosity |
|---|---|
| Comparative Example 1 | 10 % or more |
| Comparative Example 2 | Unevaluatable |
| Comparative Example 3 | 10 % or more |
| Comparative Example 4 | 10 % or more |
| Comparative Example 5 | 10 % or more |
| Example 6 | Less than 10 % |
| Example 7 | Less than 10 % |
| Example 8 | Less than 10 % |
| Example 9 | Less than 10 % |
| Example 10 | Less than 10 % |

Referring to Table 2, it can be seen that the composition of the comparative example shows a viscosity change with time of 10 % or more. In particular, in the case of Comparative Example 2, evaluation was impossible due to aggregation caused by hydrogen binding due to OH in the ligand. In the case of Comparative Examples 4 and 5, the viscosity increased by 10 % or more due to the influence of the crosslinkable moiety in the ligand.

In the case of the composition of the examples, because the end portion of the ligand includes phenyl, benzyl, and butyl groups, the composition was suitable for dispersion in 1,6-hexanediol diacrylate, and thus, the change in viscosity of the composition over time was smaller than that of the composition of the comparative examples.

### Light resistance evaluation

### Comparative Example 6

The composition of Comparative Example 1 was spin-coated on a glass to a thickness of 10 µm and exposed to UV (360 nmₘₐₓ) to prepare a color conversion layer specimen.

### Comparative Example 7

An attempt to spin-coat a color conversion layer using the quantum dot composition of Comparative Example 2 was made, but aggregation occurred, thereby making it impossible to form a coating film.

### Comparative Examples 8 to 10

A specimen was prepared in substantially the same manner as in Comparative Example 6, except that the quantum dot compositions of Comparative Examples 3 to 5 were used for each of the color conversion layers.

### Examples 11 to 15

A specimen was prepared in substantially the same manner as in Comparative Example 6, except that the quantum dot compositions of Examples 6 to 10 were used for each of the color conversion layers.

In order to evaluate the characteristics of the color conversion layers manufactured in Comparative Examples 6 and 8 to 10 and Examples 11 to 15, the color conversion layers were exposed to light of 100,000 nit high-intensity blue LED backlight that emits more than 10 times the actual usage environment for 500 hours. The results are shown in Table 3.

The efficiency (i.e., light conversion efficiency retention rate) was measured using a measurement device C9920-2-12 manufactured by Hamamatsu Photonics Inc.

**Table 3**

| | Ligand | Light conversion efficiency retention rate (%)¹ |
|---|---|---|
| Comparative Example 6 | Compound 101 | X |
| Comparative Example 7 | Compound 102 | Unevaluatable |
| Comparative Example 8 | Compound 103 | X |
| Comparative Example 9 | Compound 104 | X |
| Comparative Example 10 | Compound 105 | X |
| Example 11 | Compound 1 | O |
| Example 12 | Compound 2 | O |
| Example 13 | Compound 3 | O |
| Example 14 | Compound 4 | O |
| Example 15 | Compound 5 | O |

| | | |
|---|---|---|
| ¹ The specimen was exposed to light of 100,000 nit high-intensity blue LED backlight for 500 hours. The light conversion efficiency is maintained at 90 % or more: O The light conversion efficiency is maintained at less than 90 %: X | | |

From Table 3, it can be seen that the color conversion layer of Examples 11 to 15 have better light conversion efficiency retention rate than the color conversion layer of Comparative Examples 6 and 8-10, and thus, the light resistance (light stability) of the color conversion layer of Example 11 to 15 is much improved. Specifically, in the case of Examples 13 to 15, it was confirmed that the light conversion efficiency retention rate was improved by about 14% compared to that of Comparative Example 1. Though not to further limit the claims in any way, the presence of two thiol groups in the binding portion provides a greater binding interaction between the ligand compound and the quantum dot.

In Comparative Examples 6 and 8, although the ligand end portion includes a hydrophobic methyl group, the relatively small methyl group does not provide a sufficient hydrophobic environment to achieve an acceptable dispersion of the quantum dots in the 1,6-hexanediol diacrylate, thereby causing the poor results.

The presence of a radical stabilizing group or a group with crosslinking functionality in the end portion of a (ligand) compound may lead to an observed decrease in light resistance (light stability).

The synthesis of the compound, the quantum dots coordinated with the compound, the composition including the quantum dots, and the color conversion layer formed from the composition according to embodiments of the present disclosure are described herein.

Moreover, one of ordinary skill in the art may manufacture a light-emitting device using the quantum dots described herein in an emission layer or an electronic apparatus using the quantum dots in a color conversion layer and/or a color filter.

## Claims

1. A compound comprising:
a binding portion including a dithio C₁-C₁₆ alkyl moiety;
an end portion including an unsubstituted C₆-C₄₀ aryl group, an unsubstituted C₃-C₅₀ heteroaryl group, an unsubstituted C₂-C₁₀ alkyl group, or an unsubstituted C₇-C₅₀ aryl alkyl group; and
a hydrophilic linker including oxygen, the hydrophilic linker connecting the binding portion and the end portion,
wherein the binding portion and the hydrophilic linker are connected by an ester linkage.

2. The compound of claim 1, wherein
an alkyl of the dithio C₁-C₁₆ alkyl moiety is a linear or branched structure.

3. The compound of claim 1, wherein
one thiol group of the dithio C₁-C₁₆ alkyl moiety is positioned at a terminal carbon of the dithio C₁-C₁₆ alkyl moiety.

4. The compound of claim 1, wherein two to five carbons present between two thiol groups of the dithio C₁-C₁₆ alkyl moiety.

5. The compound of claim 1, wherein
the hydrophilic linker includes one or more ethylene glycol units, one or more propylene glycol units, or a combination thereof.

6. The compound of claim 5, wherein
a number of the one or more ethylene glycol units, or a number of the one or more propylene glycol units, are each independently 1 to 10.

7. The compound of claim 1, wherein
the unsubstituted C₆-C₄₀ aryl group includes a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, an ovalenyl group, or any combination thereof.

8. The compound of claim 1, wherein
the unsubstituted C₂-C₁₀ alkyl group includes an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or any combination thereof.

9. The compound of claim 1 represented by Formula 1, 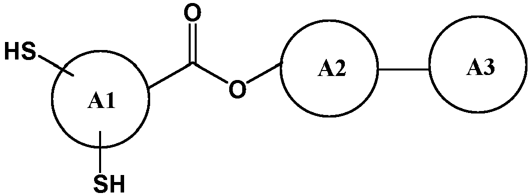 wherein, in Formula 1,
A1 is a C₁-C₁₆ alkyl moiety,
A2 comprises 1 to 6 ethylene glycol units, 1 to 6 propylene glycol units, or a combination thereof; and
A3 is an unsubstituted C₃-C₁₀ alkyl group, an unsubstituted C₆-C₂₀ aryl group, or an unsubstituted C₇-C₃₀ aryl alkyl group.

10. The compound of claim 1, wherein
the compound includes one of the following compounds:

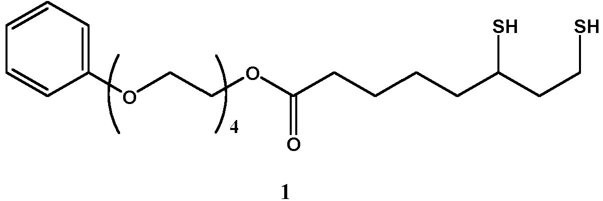

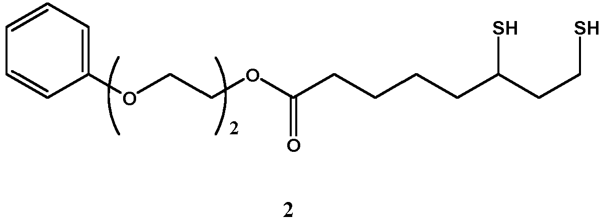

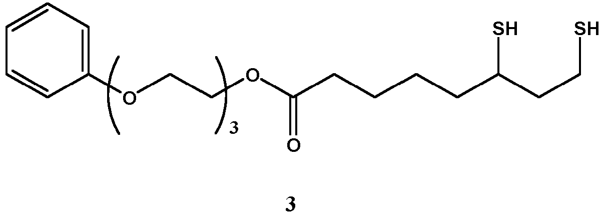

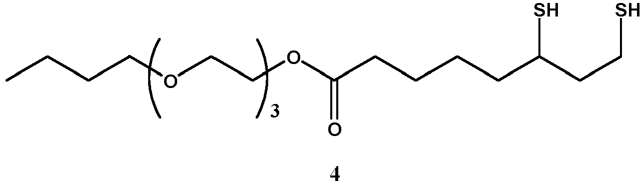

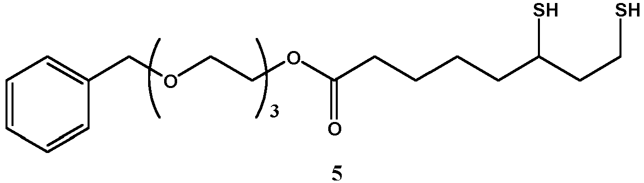

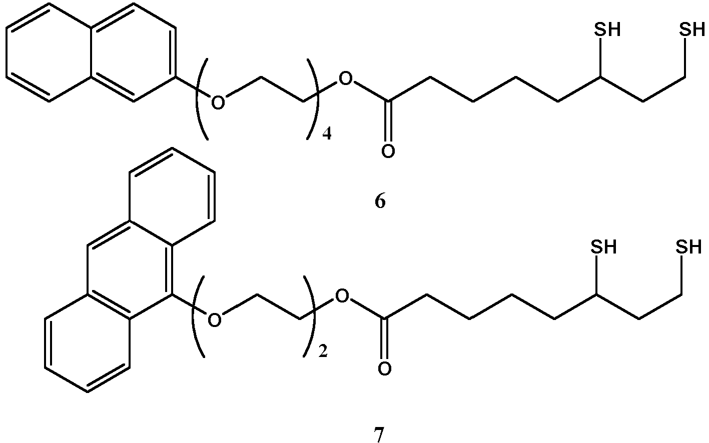

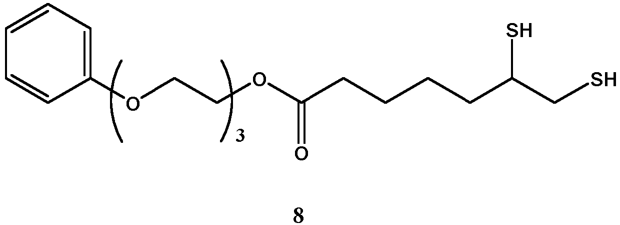

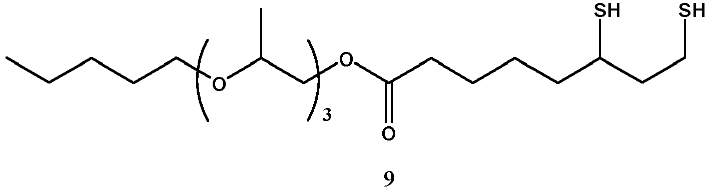

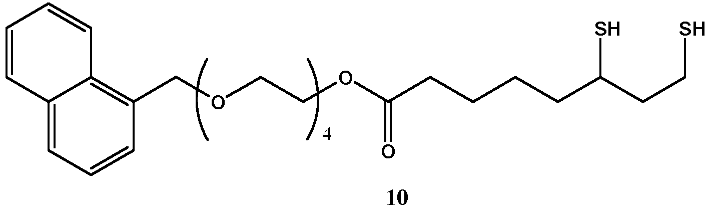

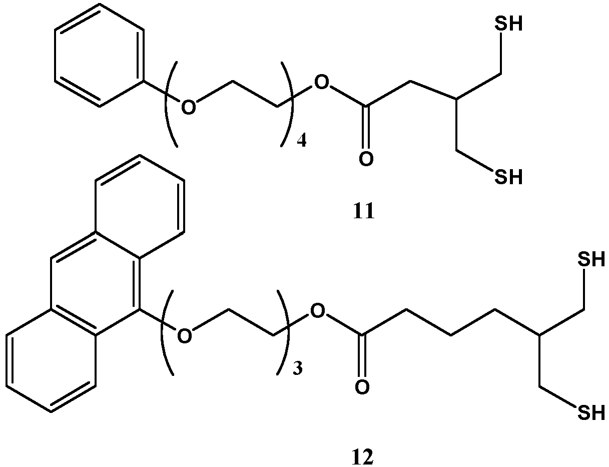

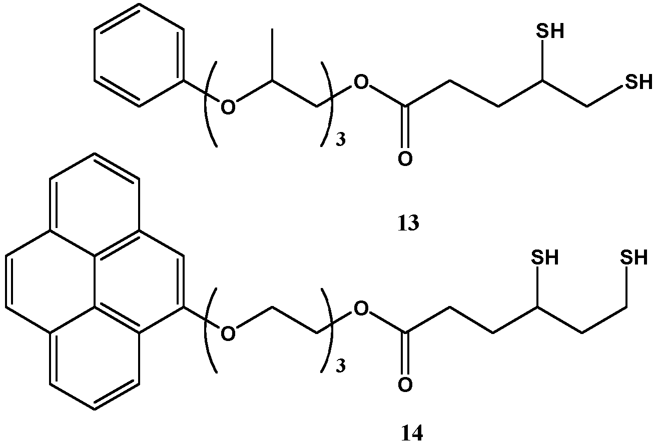

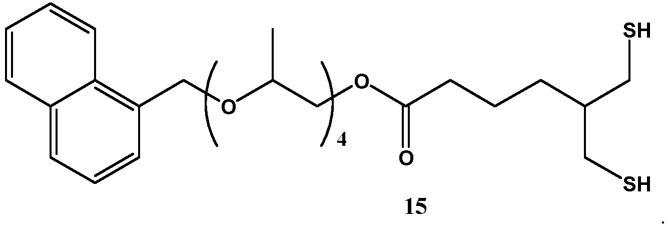

11. A quantum dot coordinated with the compound of claim 1.

12. A composition comprising the quantum dot of claim 11; and a crosslinking monomer.

13. The composition of claim 12, wherein
the composition further comprises a photoinitiator.

14. The composition of claim 12, wherein
the crosslinking monomer is an acrylic monomer.

15. The composition of claim 12, wherein
the quantum dot has a core-shell structure comprising
a core comprising a semiconductor compound and
a shell comprising an oxide of a metal, a metalloid or a non-metal, a semiconductor compound, or a combination thereof.

16. The composition of claim 15, wherein
the semiconductor compound comprises: a Group II-VI semiconductor compound; a Group III-V semiconductor compound; a Group III-VI semiconductor compound; a Group I-III-VI semiconductor compound; a Group IV-VI semiconductor compound; a Group IV element or compound; or any combination thereof, and
the oxide of the metal, the metalloid, or the non-metal each independently includes SiO₂, Al₂O₃, TiO₂, ZnO, MnO, Mn₂O₃, Mn₃O₄, CuO, FeO, Fe₂O₃, Fe₃O₄, CoO, Co₃O₄, NiO, MgAl₂O₄, CoFe₂O₄, NiFe₂O₄, CoMn₂O₄, or any combination thereof.

17. The composition of claim 15, wherein
the semiconductor compound comprises CdS, CdSe, CdTe, ZnS, ZnSe, ZnTe, ZnO, HgS, HgSe, HgTe, MgSe, MgS, CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, CdZnSeS, CdZnSeTe, CdZnSTe, CdHgSeS, CdHgSeTe, CdHgSTe, HgZnSeS, HgZnSeTe, HgZnSTe, GaN, GaP, GaAs, GaSb, AlN, AlP, AlAs, AlSb, InN, InP, InAs, InSb, GaNP, GaNAs, GaNSb, GaPAs, GaPSb, AINP, AlNAs, AlNSb, AlPAs, AlPSb, InGaP, InNP, InAlP, InNAs, InNSb, InPAs, InPSb, GaAlNP, GaAINAs, GaAINSb, GaAlPAs, GaAlPSb, GalnNP, GalnNAs, GaInNSb, GalnPAs, GalnPSb, InAlNP, InAlNAs, InAlNSb, InAlPAs, InAlPSb, InZnP, InGaZnP, InAlZnP, GaS, GaSe, Ga₂Se₃, GaTe, InS, InSe, In₂S₃, In₂Se₃, InTe, InGaS₃, InGaSe₃, AgInS, AgInS₂, CulnS, CuInS₂, CuGaO₂, AgGaO₂, AgAlO₂, SnS, SnSe, SnTe, PbS, PbSe, PbTe, SnSeS, SnSeTe, SnSTe, PbSeS, PbSeTe, PbSTe, SnPbS, SnPbSe, SnPbTe, SnPbSSe, SnPbSeTe, SnPbSTe, Si, Ge, SiC, SiGe, or any combination thereof.

18. The composition of claim 15, wherein
the semiconductor compound included in the shell comprises CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, or any combination thereof.

19. The composition of claim 12, wherein
the viscosity of the composition at 25 °C is 5 centipoise to 80 centipoise.

20. An electronic apparatus comprising: a light-emitting device including a first electrode, a second electrode facing the first electrode, and an interlayer arranged between the first electrode and the second electrode and including an emission layer;
a thin-film transistor including a source electrode and a drain electrode; and
a color conversion layer and/or a color filter; wherein
the first electrode of the light-emitting device is electrically connected to the source electrode or the drain electrode of the thin-film transistor, and
the emission layer, the color conversion layer, and/or the color filter includes a layer manufactured using the composition of claim 12.
